# EUROPÄISCHE PATENTSCHRIFT

(11) **EP 3 541 438 B1**
(45) Veröffentlichungstag und Bekanntmachung des Hinweises auf die Patenterteilung: **28.12.2022**
(21) Anmeldenummer: 17808806.8
(22) Anmeldetag: 15.11.2017
(51) Int. Cl.: A61L 27/12, A61L 27/46, A61L 31/14

(54) **IMPLANTAT UND EIN KIT ZUM BEHANDELN EINES KNOCHENDEFEKTS**
IMPLANT AND KIT FOR TREATING A BONE DEFECT
IMPLANT ET KIT DE TRAITEMENT D'UN DÉFAUT OSSEUX

(30) Priorität: 16.11.2016 DE 102016222603
(43) Veröffentlichungstag der Anmeldung: 25.09.2019
(73) Patentinhaber: Aesculap AG, 78532 Tuttlingen (DE)
(72) Erfinder: ABELE, Wolfgang, 78532 Tuttlingen (DE); HETTICH, Georg, 79117 Freiburg (DE); KÖNIG, Silke, 78628 Rottweil (DE)
(74) Vertreter: Patentanwälte Ruff, Wilhelm, Beier, Dauster & Partner mbB
(86) Internationale Anmeldenummer: PCT/EP2017/079312
(87) Internationale Veröffentlichungsnummer: WO 2018/091524

(56) Entgegenhaltungen:
- WO-A1-2012/061024
- WO-A1-2016/184699
- DE-A1-102012 213 246
- US-A1- 2015 127 106

## Beschreibung

### ANWENDUNGSGEBIET UND STAND DER TECHNIK

Die Erfindung betrifft ein Implantat sowie einen Kit zum Behandeln eines Knochendefekts.

Große Knochendefekte am Azetabulum stellen bei Revisionsoperationen am Hüftgelenk ein großes Problem dar. Bei einer Hüftrevisionsoperation werden hauptsächlich die drei nachfolgend genannten Ziele verfolgt.

Zunächst soll das ursprüngliche Gelenkzentrum wiederhergestellt werden.

Weiterhin wird eine stabile Fixierung eines zum Behandeln eines Knochendefekts eingesetzten Implantats angestrebt. Bei der Fixierung des Implantats unterscheidet man allgemein zwischen einer sogenannten Primärstabilität und einer sogenannten Sekundärstabilität.

Unter Primärstabilität wird die auf Reibung oder Befestigungselementen, wie beispielsweise Knochenschrauben, beruhende Fixierung des Implantats in den ersten Wochen nach der Operation verstanden. Unter der Sekundärstabilität wird die auf knöchernen Verwachsungen beruhende Fixierung des Implantats verstanden. Die Sekundärstabilität setzt in der Regel etwa einen Monat nach dem operativen Eingriff ein und kann bis zu mehrere Jahre andauern.

Schließlich wird eine sogenannte biologische Rekonstruktion der knöchernen Defekte angestrebt. Unter einer biologischen Rekonstruktion wird der Wiederaufbau eines Knochendefekts mit körpereignem Knochen verstanden.

Zum Behandeln von Knochendefekten haben sich in der Praxis die beiden nachfolgend beschriebenen Optionen durchgesetzt.

Die eine Option sieht die Verwendung von metallischen Augmentaten, beispielsweise in Form von metallischen, porösen Strukturen, vor. Die Verwendung von metallischen Augmentaten hat den Vorteil, dass sich das ursprüngliche Gelenkzentrum in der Regel gut wiederherstellen lässt. Obendrein ist eine gute Fixierung des Implantats erzielbar. Nachteilig ist jedoch, dass metallische Augmentate unverändert im Körper bestehen bleiben, eine biologische Rekonstruktion knöcherner Defekte mithin nicht stattfindet. Hinzu kommt, dass bei einer (erneuten) Revision das gesamte Augmentat ausgewechselt werden muss, was in der Regel eine Defektvergrößerung zur Folge hat. Dies gilt insbesondere im Falle des Auftretens von knöchernen Verwachsungen mit dem metallischen Augmentat. Ein weiterer Nachteil besteht darin, dass metallische Augmentate nicht formbar sind. Dies wiederum kann zur Folge haben, dass der Defekt an das Augmentat angepasst werden muss, wodurch der Defekt gewöhnlich ebenfalls vergrößert wird. Außerdem ist von Nachteil, dass metallische Augmentate nicht bearbeitbar sind, wodurch im Falle einer (erneuten) Revision ein Aufbau auf das Augmentat nicht möglich ist.

Die zweite Option zur Fixierung von Implantaten, welche zum Behandeln von Knochendefekten verwendet werden, sieht die Verwendung von Knochenchips oder -spänen vor. Knochenchips bzw. -späne haben den Vorteil, dass sie eine Rekonstruktion knöcherner Defekte erlauben. Auch die Wiederherstellung des Rotationszentrums bereitet in der Regel keine Probleme. Nachteilig ist jedoch die chirurgische Handhabung von Knochenchips bzw. -spänen. So muss das Knochenmaterial üblicherweise während der Operation bereitgestellt werden. Hierzu wird ein allogener Femurkopf aufgetaut und zerkleinert. Erschwert wird die Handhabung durch die unregelmäßigen Formen der Knochenpartikel. Es bedarf daher einiger Erfahrung auf Seiten des Operateurs, um diese Knochenfüllmaterialien richtig einzusetzen. Da die Knochenbanken zudem strenge Auflagen erfüllen müssen, ist die Bereitstellung von Knochenchips bzw. -spänen insgesamt sehr aufwändig und teuer. Ein weiterer Nachteil besteht in dem potenziellen Infektionsrisiko. Schließlich ist von Nachteil, dass sich Knochenchips bzw. -späne allgemein durch eine lediglich begrenzte mechanische Belastbarkeit (geringe Primärstabilität) auszeichnen. Zudem besteht die Gefahr der Resorption, ohne dass ein Knochenwachstum eingesetzt oder stattgefunden hat. Mit anderen Worten besteht ein gewisses Risiko, dass sich keine Sekundärstabilität erzielen lässt.

Aus der US 8,562,613 B2 ist ein Kit zur Behandlung von Knochendefekten mit einer Mischung aus einem osteokonduktiven Material und einem osteoinduktiven Material sowie einem porösen Container bekannt.

Gegenstand der EP 0 764 008 B1 ist eine Vorrichtung zur Verwendung bei der Stabilisierung eines spinalen Bewegungssegments mit einem flexiblen Beutel, wobei der Beutel ein biologisches Füllmaterial zur Förderung einer Knochen- oder Faserverwachsung enthalten kann.

Die EP 1 408 888 B1 offenbart ein System zur Korrektur von Wirbelkompressionsfrakturen, welches einen porösen Beutel sowie ein Füllwerkzeug aufweist, wobei das Füllwerkzeug dazu ausgebildet ist, um ein Knochenfüllmaterial unter Druck in den porösen Beutel zu injizieren.

Aus der WO 2012/061024 A1 ist ein implantierbarer Container bekannt, welcher wenigstens teilweise demineralisierte und osteoinduktive Knochenpartikel enthält.

Ein Knochenimplantat mit einem synthetischen Knochenersatzmaterial und einer resorbierbaren netzförmigen Umhüllung ist aus der US 2015/0127106 A1 bekannt.

Ein Kit zur Behandlung von Knochendefekten, das oligopodenförmige Stützkörper sowie ein Bindemittel aufweist, ist aus der DE 10 2012 213 246 A1 bekannt.

Die Publikation "Bone Regeneration by the Combined Use of Tetrapod-Shaped Calcium Phosphate Granules with Basic Fibroblast Growth Factor-Binding Ion Complex Gel in Canine Segmental Radial Defects (J. Vet. Med. Sci. 76(7):955-961, 2014)" von Honnami et al. beschäftigt sich mit einer Kombination von tetrapodenförmigem Granulat aus alpha-Tricalciumphosphat und einem osteoinduktiven Gel.

### AUFGABE UND LÖSUNG

Aufgabe der Erfindung ist es, ein Implantat bereitzustellen, welches sich zum Behandeln eines Knochendefekts, insbesondere periprothetischen Knochendefekts, eignet und die einleitend genannten Nachteile, insbesondere im Zusammenhang einer Hüftrevisionsoperation, vermeidet oder wenigstens weitgehend vermeidet. Das Implantat soll eine ausreichende mechanische Stabilität wie Primär- und/oder Sekundärstabilität und gegebenenfalls eine biologische Rekonstruktion eines knöchernen Defekts sowie eine möglichst einfache Handhabung ermöglichen. Insbesondere soll das Implantat dazu geeignet sein, alle drei im Zusammenhang einer Hüftrevisionsoperation genannten Anforderungen (Primärstabilität, Sekundärstabilität und biologische Rekonstruktion) gerecht zu werden.

Diese Aufgabe wird gelöst durch ein Implantat mit den Merkmalen gemäß unabhängigem Anspruch 1. Bevorzugte Ausführungsformen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Implantat, vorzugsweise zum Behandeln und/oder biologischen Rekonstruieren, insbesondere Auskleiden und/oder Abdichten und/oder Unterfüttern und/oder wenigstens teilweisen Auffüllen, eines Knochendefekts. Bei dem Implantat handelt es sich vorzugsweise um ein chirurgisches Implantat.

Das Implantat weist Folgendes auf oder besteht aus Folgendem:
- osteokonduktive Stützkörper und
- eine Einbringhilfe.

Das Implantat zeichnet sich dadurch aus, dass die Einbringhilfe zum Einbringen der osteokonduktiven Stützkörper in einen Knochendefekt und zum Zusammenhalten, insbesondere dauerhaften oder vorübergehenden Zusammenhalten, der osteokonduktiven Stützkörper, vorzugsweise während und/oder nach deren Einbringen in einen Knochendefekt, ausgebildet ist.

Der Ausdruck "Knochendefekt" bedeutet im Sinne der vorliegenden Erfindung einen durch Verlust von Knochengewebe, insbesondere Gelenksknochengewebe, vorzugsweise Hüftgelenks- oder Kniegelenksknochengewebe, oder von Wirbelkörpergewebe betroffenen Knochenbereich, insbesondere Gelenksknochenbereich, vorzugsweise Hüftgelenks- oder Kniegelenksknochenbereich, oder Wirbelkörperbereich. Der Knochenverlust kann die Folge einer Knochenfraktur, eines Knochentraumas, einer Knochenerkrankung wie Tumorerkrankung oder einer chirurgischen Intervention/Reintervention, insbesondere einer Revision nach einer totalen Hüft- oder Kniearthroplastik, sein. Bevorzugt bedeutet der Ausdruck "Knochendefekt" im Sinne der vorliegenden Erfindung einen periprothetischen Knochendefekt, d.h. einen durch periprothetischen Knochengewebsverlust, insbesondere aufgrund von mechanischer Überlastung und/oder abrieb-induzierter Osteolyse und/oder Implantatmigration, betroffenen Knochenbereich.

Vorzugsweise handelt es sich bei dem Knochendefekt um einen Gelenksknochendefekt, insbesondere Kniegelenksknochendefekt oder Hüftgelenksknochendefekt, vorzugsweise Azetabulumdefekt.

Weiterhin kann der Ausdruck "Knochendefekt" im Sinne der vorliegenden Erfindung einen menschlichen Knochendefekt oder einen tierischen Knochendefekt bedeuten.

Unter dem Ausdruck "tierischer Knochendefekt" soll im Sinne der vorliegenden Erfindung der Knochendefekt eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "Stützkörper" bedeutet im Sinne der vorliegenden Erfindung Körper, insbesondere regelmäßig und/oder unregelmäßig geformte Körper, welche dazu ausgebildet sind, in einem zu behandelnden Knochendefekt üblicherweise auftretenden Kräften ohne Deformation oder Zerstörung, wenigstens jedoch ohne wesentliche Deformation oder Zerstörung, zu widerstehen und mithin lasttragende Funktionen zu übernehmen. Daher können die Stützkörper im Sinne der vorliegenden Erfindung auch als osteokonduktive, lasttragende Stützkörper bezeichnet werden.

Der im Zusammenhang mit den Stützkörpern verwendete Ausdruck "osteokonduktiv" bedeutet im Sinne der vorliegenden Erfindung die Fähigkeit der Stützkörper zur Ausbildung einer dreidimensionalen Struktur, insbesondere Leitstruktur, oder einer dreidimensionalen Matrix, insbesondere Leitmatrix, welche ein Einwachsen von Knochengewebe, insbesondere Knochenneugewebe, begünstigt.

Der Ausdruck "Einbringhilfe" bedeutet im Sinne der vorliegenden Erfindung ein Mittel, welches dazu ausgebildet ist, osteokonduktive Stützkörper in einen Knochendefekt einzubringen und osteokonduktive Stützkörper zusammenzuhalten, insbesondere dauerhaft oder vorübergehend, vorzugsweise während und/oder nach Einbringen der osteokonduktiven Stützkörper in einen Knochendefekt. Die Einbringhilfe kann im Sinne der vorliegenden Erfindung daher auch als Einbringmittel bezeichnet werden.

Der Ausdruck "osteoaktiv und in vivo abbaubar/in vivo resorbierbar" bedeutet im Sinne der vorliegenden Erfindung osteoaktiv und in vivo abbaubar oder osteoaktiv und in vivo resorbierbar.

Der Ausdruck "osteoaktiv" bezieht sich im Sinne der vorliegenden Erfindung vorzugsweise auf ein sogenanntes osteogenes Material, d.h. ein Material, welches das Wachstum von (bereits vorhandenem) Knochengewebe stimuliert und/oder verstärkt, und/oder auf ein sogenanntes osteoinduktives Material, d.h. ein Material, welches die Neubildung (sogenannte Neogenese) von Knochengewebe stimuliert und/oder verstärkt. Insbesondere kann sich der Ausdruck "osteoaktiv" auf ein Material beziehen, welches osteoaktive und osteoinduktive Eigenschaften besitzt.

Der Ausdruck "in vivo abbaubar" bezieht sich im Sinne der vorliegenden Erfindung auf einen Stoff oder ein Material, welcher bzw. welches in einem menschlichen oder tierischen Körper, insbesondere unter der Einwirkung von Enzymen, metabolisiert werden kann. Der Abbau des Stoffs bzw. Materials kann dabei vollständig bis zur Mineralisierung, d.h. Freisetzung von chemischen Elementen und deren Einbau in anorganische Verbindungen, wie beispielsweise Kohlendioxid, Sauerstoff und/oder Ammoniak, verlaufen oder auf der Stufe von abbaustabilen Zwischen- oder Transformationsprodukten stehen bleiben.

Unter dem Ausdruck "tierischer Körper" soll im Sinne der vorliegenden Erfindung der Körper eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "in vivo resorbierbar" bezieht sich im Sinne der vorliegenden Erfindung auf einen Stoff oder ein Material, welcher bzw. welches in einem menschlichen oder tierischen Körper durch lebende Zellen oder lebendes Gewebe, wie beispielsweise Nieren, aufgenommen werden kann, ohne dass ein Abbau oder ein nennenswerter Abbau des Materials stattfindet.

Unter dem Ausdruck "Umhüllung" soll im Sinne der vorliegenden Erfindung ein Gebilde oder Konstrukt verstanden werden, welches dazu ausgebildet ist, (wenigstens) die osteokonduktiven Stützkörper vollständig zu umgeben oder zu umschließen. Hierzu weist die Umhüllung bevorzugt einen Hohlraum auf, welcher (wenigstens) mit den osteokonduktiven Stützkörpern wenigstens teilweise, bevorzugt nur teilweise, befüllbar oder befüllt ist.

Die vorliegende Erfindung zeichnet sich insbesondere durch die folgenden Vorteile aus:
- Bei gutem Heilungsverlauf können die osteokonduktiven Stützkörper mit besonderem Vorteil von körpereigenem Knochen integriert oder zellulär umgebaut werden. Dadurch kann eine biologische Rekonstruktion eines Knochendefekts gewährleistet werden. Bei Patienten mit langsamem Knochenwachstum bleiben die osteokonduktiven Stützkörper vorzugsweise erhalten, wenigstens solange bis eine ausreichende Sekundärstabilität erzielt worden ist. Dies ist insbesondere im Hinblick auf ältere Patienten, bei welchen ein Knochenwachstum häufig überhaupt nicht mehr stattfindet, von Vorteil.
- Ein weiterer Vorteil besteht darin, dass die osteokonduktiven Stützkörper in einem verdichteten, bevorzugt impaktierten (eingeklemmten oder verkeilten), Zustand als Platzhalter und/oder Leitstruktur ein Einwachsen von Knochengewebe, insbesondere neuem Knochengewebe, in das Implantat und insbesondere in einen zu behandelnden Knochendefekt bewirken können.
- Ein weiterer Vorteil besteht darin, dass das Implantat, insbesondere wenn die osteokonduktiven Stützkörper in einem verdichteten, bevorzugt impaktierten, Zustand vorliegen, dauerhaft und vor allem homogen belastet werden kann. Eine homogene Implantatbelastung stellt eine Grundvoraussetzung für Knochenwachstum dar. Beispielsweise kann das Implantat, insbesondere mit verdichtetet, bevorzugt impaktiert, vorliegenden osteokonduktiven Stützkörpern, dauerhaft mit einer Druckbelastung von bis zu 10 MPa beaufschlagt werden. Dabei kann eine durch Verdichten, insbesondere Impaktieren, erzeugte und von den osteokonduktiven Stützkörpern aufgebaute Struktur mit besonderem Vorteil eine elastische Verformung, insbesondere von 5 % bis 15 %, und ein geringes E-Modul, insbesondere von 50 MPa bis 300 MPa, aufweisen.

- Im Gegensatz zu gattungsgemäßen Implantaten, insbesondere metallischen Augmentaten, bei welchen lediglich Zwischenräume an Implantatrandzonen mechanisch belastet werden, können in einem verdichteten Zustand der osteokonduktiven Stützkörper vorhandene Hohl- und/oder Zwischenräume aufgrund des geringen E-Moduls auch innerhalb einer kompletten Defektauffüllung, d.h. homogen, mechanisch belastet werden (Mikrobewegungen). Dies wiederum bewirkt eine Stimulierung und/oder Verstärkung von Knochenwachstum, insbesondere von Knochenneubildung. Insgesamt ist dadurch eine homogene Verknöcherung des gesamten Knochendefekts erzielbar.
- Durch die Einbringhilfe wird mit besonderem Vorteil das Einbringen der osteokonduktiven Stützkörper in einen zu behandelnden Knochendefekt und damit insbesondere die operative Handhabung des Implantats für einen Anwender, vorzugsweise Chirurgen, erleichtert.
- Ein weiterer Vorteil der Einbringhilfe besteht insbesondere darin, dass sie eine unerwünschte Dislokation oder Verbreitung der osteokonduktiven Stützkörper innerhalb eines zu behandelnden Knochendefekts, insbesondere während eines operativen Eingriffs, verhindert, indem sie die osteokonduktiven Stützkörper zusammenhält. Mit anderen Worten ist mittels der Einbringhilfe eine Ortsbindung der osteokonduktiven Stützkörper innerhalb eines zu behandelnden Knochendefekts erzielbar, wodurch deren Osteokonduktivität sowie die oben im Zusammenhang der Stützkörper genannten Vorteile besonders gut zur Entfaltung kommen können. Beispielsweise ermöglicht die Einbringhilfe ein möglichst vollständiges Verdichten, insbesondere Impaktieren, der Stützkörper nach Einbringen in einen Knochendefekt, ohne dass sich wenigstens ein Teil der Stützkörper während des Verdichtens, insbesondere Impaktierens, unkontrolliert innerhalb des Knochendefekts verteilt. Dadurch lässt sich mit den Stützkörpern eine besonders wirkungsvolle osteokonduktive Leitstruktur aufbauen, was wiederum wesentlich für eine ausreichende Sekundärstabilität sowie insbesondere für eine biologische Rekonstruktion ist.
- Das erfindungsgemäße Implantat eignet sich insbesondere zur biologischen Rekonstruktion von großen Knochendefekten, insbesondere von Knochendefekten, welche eine Belastungsstabilität von bis zu 10 MPa Druck erfordern.

In einer bevorzugten Ausführungsform weisen die osteokonduktiven Stützkörper Apatit und/oder Tricalciumphosphat auf oder bestehen aus Apatit und/oder Tricalciumphosphat. Die vorliegende Erfindung beruht insbesondere auf dem überraschenden Befund, dass die oben im Zusammenhang der osteokonduktiven Stützkörper genannten Vorteile besonders stark zur Geltung kommen, wenn die Stützkörper Apatit und/oder Tricalciumphosphat aufweisen oder aus Apatit und/oder Tricalciumphosphat bestehen.

In einer weiteren Ausführungsform weisen die osteokonduktiven Stützkörper Apatit auf oder bestehen aus Apatit.

In einer weiteren Ausführungsform handelt es sich bei dem Apatit um einen nicht in vivo abbaubaren/nicht in vivo resorbierbaren Apatit. Dadurch kann selbst bei Patienten, bei welchen nicht mehr mit einem (ausreichenden) Knochenwachstum zu rechnen ist, eine ausreichende Sekundärstabilität erzielt werden. Dies ist insbesondere im Hinblick auf die Behandlung von Knochendefekten älterer Patienten von Vorteil.

In einer weiteren Ausführungsform handelt es sich bei dem Apatit um einen in vivo abbaubaren/in vivo resorbierbaren Apatit, vorzugsweise um einen langsam in vivo abbaubaren/langsam in vivo resorbierbaren Apatit.

In einer weiteren Ausführungsform weist der Apatit eine in vivo Abbauzeit (Degradationszeit) oder eine in vivo Resorptionszeit von 6 Monate bis 30 Jahre, insbesondere 1 Jahr bis 20 Jahre, bevorzugt 4 Jahre bis 10 Jahre, auf. Die in diesem Absatz offenbarten Abbau- bzw. Resorptionszeiten sind insbesondere im Hinblick auf die Behandlung von Patienten mit einem langsamen Knochenwachstum von Vorteil.

Der Apatit liegt in einer weiteren Ausführungsform in kristalliner Form vor. Durch eine hohe Kristallinität kann eine hohe Festigkeit erreicht werden. Durch eine niedrige Kristalinität kann eine gute und/oder schnelle Abbaubarkeit erreicht werden.

In einer weiteren Ausführungsform handelt es sich bei dem Apatit um einen mikrokristallinen Apatit, d.h. um einen Apatit mit Kristalliten, welche wenigstens eine Abmessung oder Dimension im Mikrometerbereich, insbesondere in einem Bereich von > 0.5 µm, insbesondere 0.6 µm bis 500 µm, bevorzugt 0.6 µm bis 100 µm, aufweisen. Bei der wenigstens einen Abmessung bzw. Dimension kann es sich insbesondere um die Länge und/oder Breite (Dicke bzw. Höhe) und/oder den Durchmesser, insbesondere mittleren Durchmesser, der Kristallite handeln.

Grundsätzlich kann es sich bei dem Apatit jedoch auch um einen makrokristallinen Apatit handeln.

In einer weiteren Ausführungsform handelt es sich bei dem Apatit um einen nanokristallinen Apatit, d.h. um einen Apatit mit Kristalliten, welche wenigstens eine Abmessung oder Dimension im Nanometerbereich, insbesondere in einem Bereich von 0.1 nm bis 500 nm, bevorzugt 0.1 nm bis 100 nm, aufweisen. Bei der wenigstens einen Abmessung bzw. Dimension kann es sich insbesondere um die Länge und/oder Breite (Dicke bzw. Höhe) und/oder den Durchmesser, insbesondere mittleren Durchmesser, der Kristallite handeln.

In einer weiteren Ausführungsform liegt der Apatit in amorpher Form vor. Dadurch kann eine besonders gute und/oder schnelle Resorption erreicht werden.

In einer weiteren Ausführungsform handelt es sich bei dem Apatit um einen phasenreinen Apatit. Unter dem Ausdruck "phasenrein" soll insbesondere phasenrein im Sinne einer einschlägigen Vorschrift, vorzugsweise nach ASTM F1185, verstanden werden.

In einer weiteren Ausführungsform weist der Apatit eine Porosität kleiner 50 %, insbesondere kleiner 20 %, vorzugsweise kleiner 15 %, auf. Durch eine geringe Porosität kann eine hohe mechanische Stabilität erreichen werden.

In einer weiteren Ausführungsform ist der Apatit nicht porös ausgebildet.

Insbesondere können die Stützkörper eine Kombination von porösen Apatiten und/oder von Apatiten mit unterschiedlicher Porosität und/oder von nichtporösen Apatiten aufweisen, insbesondere wenn die Stützkörper nach einem additiven Fertigungsverfahren hergestellt sind.

In einer weiteren Ausführungsform handelt es sich bei dem Apatit um natürlich vorkommenden Apatit oder um einen aus natürlichem Apatit gewonnenen Apatit.

In einer weiteren Ausführungsform handelt es sich bei dem Apatit um einen synthetischen, d.h. künstlichen oder artifiziellen, Apatit.

In einer weiteren Ausführungsform ist der Apatit ausgewählt aus der Gruppe bestehend aus Hydroxylapatit, Fluorapatit, Chlorapatit, Carbonat-Fluor-Apatit und Mischungen aus wenigstens zwei der genannten Apatite.

Besonders bevorzugt handelt es sich bei dem Apatit um Hydroxylapatit. Beispielsweise kann es sich bei dem Hydroxylapatit um einen vollsynthetischen, nanokristallinen und phasenreinen Hydroxylapatit handeln. Ein solcher Hydroxylapatit ist im Handel beispielsweise unter der Bezeichnung Ostim oder Nanogel kommerziell erhältlich.

In einer weiteren Ausführungsform handelt es sich bei dem Apatit um einen gesinterten Apatit.

Vorzugsweise ist der gesinterte Apatit ausgewählt aus der Gruppe bestehend aus gesinterter Hydroxylapatit, gesinterter Fluorapatit, gesinterter Chlorapatit, gesinterter Carbonat-Fluor-Apatit und Mischungen aus wenigstens zwei der genannten gesinterten Apatite.

In einer weiteren bevorzugten Ausführungsform weisen die osteokonduktiven Stützkörper Tricalciumphosphat auf oder bestehen aus Tricalciumphosphat.

In einer weiteren Ausführungsform handelt es sich bei dem Tricalciumphosphat um ein nicht in vivo abbaubares/nicht in vivo resorbierbares Tricalciumphosphat. Dadurch kann selbst bei Patienten, bei welchen nicht mehr mit einem (ausreichenden) Knochenwachstum zu rechnen ist, eine ausreichende Sekundärstabilität erzielt werden. Dies ist insbesondere im Hinblick auf die Behandlung von Knochendefekten älterer Patienten von Vorteil.

In einer weiteren Ausführungsform handelt es sich bei dem Tricalciumphosphat um in vivo abbaubares/in vivo resorbierbares Tricalciumphosphat, vorzugsweise um ein langsam in vivo abbaubares/langsam in vivo resorbierbares Tricalciumphosphat.

In einer weiteren Ausführungsform weist das Tricalciumphosphat eine in vivo Abbauzeit (Degradationszeit) oder eine in vivo Resorptionszeit von 1 Monat bis 15 Jahre, insbesondere 6 Monate bis 10 Jahre, bevorzugt 1 Jahr bis 5 Jahre, auf. Die in diesem Absatz offenbarten Abbau- bzw. Resorptionszeiten sind insbesondere im Hinblick auf die Behandlung von Patienten mit einem langsamen Knochenwachstum von Vorteil.

Das Tricalciumphosphat liegt in einer weiteren Ausführungsform in kristalliner Form vor. Durch eine hohe Kristallinität kann eine hohe Festigkeit erreicht werden. Durch eine niedrige Kristalinität kann eine gute und/oder schnelle Abbaubarkeit erreicht werden.

Bevorzugt weist das Tricalciumphosphat eine Kristallinität von 50 % bis 99 %, insbesondere 75 % bis 95 %, auf.

In einer weiteren Ausführungsform handelt es sich bei dem Tricalciumphosphat um mikrokristallines Tricalciumphosphat, d.h. um Tricalciumphosphat mit Kristalliten, welche wenigstens eine Abmessung oder Dimension im Mikrometerbereich, insbesondere in einem Bereich von > 0.5 µm, insbesondere 0.6 µm bis 500 µm, bevorzugt 0.6 µm bis 100 µm, aufweisen. Bei der wenigstens einen Abmessung bzw. Dimension kann es sich insbesondere um die Länge und/oder Breite (Dicke bzw. Höhe) und/oder den Durchmesser, insbesondere mittleren Durchmesser, der Kristallite handeln.

Grundsätzlich kann es sich bei dem Tricalciumphosphat aber auch um ein makrokristallines Tricalciumphosphat handeln.

In einer weiteren Ausführungsform handelt es sich bei dem Tricalciumphosphat um nanokristallines Tricalciumphosphat, d.h. um Tricalciumphosphat mit Kristalliten, welche wenigstens eine Abmessung oder Dimension im Nanometerbereich, insbesondere in einem Bereich von 0.1 nm bis 500 nm, bevorzugt 0.1 nm bis 100 nm, aufweisen. Bei der wenigstens einen Abmessung bzw. Dimension kann es sich insbesondere um die Länge und/oder Breite (Dicke bzw. Höhe) und/oder den Durchmesser, insbesondere mittleren Durchmesser, der Kristallite handeln.

In einer weiteren Ausführungsform liegt das Tricalciumphosphat in amorpher Form vor. Dadurch kann eine besonders gute und/oder schnelle Resorption erreicht werden.

In einer weiteren Ausführungsform handelt es sich bei dem Tricalciumphosphat um ein phasenreines Tricalciumphosphat. Unter dem Ausdruck "phasenrein" soll insbesondere phasenrein im Sinne einer einschlägigen Vorschrift, vorzugsweise nach ASTM F1088, verstanden werden.

In einer weiteren Ausführungsform weist das Tricalciumphosphat eine Porosität kleiner 50 %, insbesondere kleiner 20 %, vorzugsweise kleiner 15 %, auf.

In einer weiteren Ausführungsform ist das Tricalciumphosphat nicht porös ausgebildet.

In einer weiteren Ausführungsform handelt es sich bei dem Tricalciumphosphat um natürlich vorkommendes Tricalciumphosphat oder um ein aus natürlichem Tricalciumphosphat gewonnenes Tricalciumphosphat.

In einer weiteren Ausführungsform handelt es sich bei dem Tricalciumphosphat um ein synthetisches, d.h. künstliches oder artifizielles, Tricalciumphosphat.

Das Tricalciumphosphat ist in einer besonders bevorzugten Ausführungsform ausgewählt aus der Gruppe bestehend aus alpha-Tricalciumphosphat (a-TCP), beta-Tricalciumphosphat (β-TCP) und einer Mischung aus alpha-Tricalciumphosphat und beta-Tricalciumphosphat.

In einer weiteren Ausführungsform handelt es sich bei dem Tricalciumphosphat um gesintertes Tricalciumphosphat.

Das gesinterte Tricalciumphosphat ist vorzugsweise ausgewählt aus der Gruppe bestehend aus gesintertes alpha-Tricalciumphosphat, gesintertes beta-Tricalciumphosphat und einer Mischung aus gesintertem alpha-Tricalciumphosphat und gesintertem beta-Tricalciumphosphat.

In einer weiteren Ausführungsform weisen die osteokonduktiven Stützkörper Apatit und Tricalciumphosphat - sogenanntes biphasisches Calciumphosphat (BCP) - auf oder bestehen aus Apatit und Tricalciumphosphat. Vorzugsweise weisen die osteokonduktiven Stützkörper Hydroxylapatit und beta-Tricalciumphosphat auf oder bestehen aus Hydroxylapatit und beta-Tricalciumphosphat. Das biphasische Calciumphosphat kann insbesondere ein Verhältnis von Hydroxylapatit (HA) zu beta-Tricalciumphosphat (ß-TCP) von 80:20 bis 20:80 aufweisen. Zum Beispiel kann das biphasische Calciumphosphat aus 60% Hydroxylapatit (HA) und 40% beta-Tricalciumphosphat (β-TCP) bestehen. Insbesondere kann das biphasische Calciumphosphat aus 50% Hydroylapatit (HA) und 50% beta-Tricalciumphosphat (ß-TCP) bestehen. Um so höher der Anteil an Hydroxylapatit (HA), um so langsamer und kontrollierter ist der Abbau (Degradation) oder die Resorption. Durch Sintern können sich kompakte, hochkristalline Strukturen mit Kristallitgrößen von einigen Mikrometern ausbilden. Bezüglich weiterer Merkmale und Vorteile des Apatits sowie des Tricalciumphosphats wird auf die vorangegangenen Ausführungen Bezug genommen.

In einer weiteren Ausführungsform weisen die Stützkörper eine angeraute Oberfläche auf. Dadurch kann ein Anwachsen oder Anhaften von Knochengewebe, insbesondere an eine durch die Stützkörper gebildete osteokonduktive Leitstruktur, optimiert werden. Unter dem Ausdruck "anrauen" soll im Sinne der vorliegenden Erfindung insbesondere verstanden werden, dass eine Rauheit der Oberfläche nach einer Ausformung der Stützkörper erhöht wird, insbesondere in einem dafür vorgesehenen Herstellungsschritt. Das Anrauen kann zum Beispiel durch eine Anätzung, insbesondere mittels Phosphorsäure, geschehen. Vorzugsweise weisen die Stützkörper eine angeraute Oberfläche auf, deren Rauigkeit gegenüber einer nicht angerauten Stützkörperoberfläche um wenigstens 10 % erhöht ist. Unter dem Ausdruck "Rauheit" soll insbesondere eine Unebenheit der Oberfläche der Stützkörper verstanden werden.

In einer weiteren Ausführungsform können die Stützkörper mittels eines additiven Fertigungsverfahrens hergestellt sein.

In einer weiteren Ausführungsform weisen die Stützkörper Calciumphosphatzement auf oder bestehen aus Calciumphosphatzement. Bei dem Calciumphosphatzement kann es sich insbesondere um einen Calciumphosphatzement handeln, welcher vor einem vollständigen Aushärten einem Druck, vorzugsweise absoluten Druck, von wenigstens 2 bar unterworfen wird. Auf diese Weise kann mit besonderem Vorteil die Porosität erniedrigt werden.

In einer weiteren Ausführungsform sind die osteokonduktiven Stützkörper stoffschlüssig miteinander verbunden, insbesondere miteinander verklebt.

In einer weiteren Ausführungsform sind die osteokonduktiven Stützkörper mit einem Bindemittel beschichtet. Bei dem Bindemittel handelt es sich vorzugsweise um ein Bindemittel, welches durch Hitze oder ein Lösungsmittel, wie beispielsweise N-Methyl-Pyrrolidon (NMP) oder Aceton, angelöst werden kann. Durch die Verwendung eines derartigen Bindemittels ist es möglich, die osteokonduktiven Stützkörper durch Erhitzen und anschließendes Abkühlen oder durch Zugabe eines Lösungsmittels miteinander zu verbinden. Bei dem Bindemittel kann es sich beispielsweise um Polylactid und/oder Poly(lactid-co-Glycolid) (PLGA) handeln. Alternativ oder in Kombination kann es sich bei dem Bindemittel um ein Polysaccharid handeln, oder kann das Bindemittel ein Polysaccharid aufweisen. Das Polysaccharid kann insbesondere ausgewählt sein, aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Carboxyethylcellulose, Algininsäure, Alginate, Chitin, Chitosan, Hyaluronsäure, Dextransulfat, Heparin, Heparansulfat, Chondroitinsulfat wie Chondroitin-4-sulfat und/oder Chondroitin-6-sulfat, Dermatansulfat, Keratansulfat und Mischungen davon. Alternativ oder in Kombination kann es sich bei dem Bindemittel um ein synthetisches Polymer handeln, oder kann das Bindemittel ein synthetisches Polymer aufweisen. Das synthetische Polymer kann insbesondere ausgewählt sein aus der Gruppe bestehend aus Polyvinylalkohol, Polyethylenglykole, Ethylenoxid-Propylenoxid-Copolymere (EO-PO-Copolymere), Ethylenoxid-Propylenoxid-Blockcopolymere (EO-PO-Blockcopolymere), Acrylsäure-Homopolymere, Acrylsäure-Copolymere, Polyvinylpyrrolidon-Homopolymere, Polyvinylpyrrolidon-Copolymere und Mischungen davon.

In einer weiteren Ausführungsform sind die osteokonduktiven Stützkörper derart ausgebildet, das sie ein Verdichten, vorzugsweise Impaktieren, insbesondere ein gegenseitiges Einklemmen oder Verkeilen, der Stützkörper, beispielsweise mittels eines geeigneten Instruments wie eines Nachschlägers, begünstigen. Bezüglich entsprechend geeigneter Ausgestaltungen der osteokonduktiven Stützkörper sei auf die nachfolgenden Ausführungen verwiesen.

Die osteokonduktiven Stützkörper sind in einer weiteren Ausführungsform regelmäßig geformt, d.h. liegen gemäß einer weiteren Ausführungsform als Formkörper vor. Unter dem Ausdruck "regelmäßig geformt" sollen im Sinne der vorliegenden Erfindung insbesondere die nachfolgend beschriebenen Formen verstanden werden.

Die osteokonduktiven Stützkörper, insbesondere Formkörper, können insbesondere einen polygonen Querschnitt aufweisen. Beispielsweise können die osteokonduktiven Stützkörper, insbesondere Formkörper, einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt aufweisen.

Weiterhin können die osteokonduktiven Stützkörper, insbesondere Formkörper, unterschiedliche Querschnitte aufweisen. Bezüglich möglicher Querschnitte wird auf die im vorherigen Absatz genannten Querschnitte Bezug genommen.

Die osteokonduktive Stützkörper, insbesondere Formkörper, sind in einer weiteren Ausführungsform polyederförmig, insbesondere quader-, würfel-, tetraeder-, prismen-, pyramiden-, pyramidenstumpf- oder spatförmig, ausgebildet.

Weiterhin können die osteokonduktiven Stützkörper, insbesondere Formkörper, unterschiedlich polyederförmig ausgebildet sein. Mit anderen Worten können die osteokonduktiven Stützkörper, insbesondere Formkörper, in unterschiedlichen Polyederformen vorliegen. Bezüglich möglicher polyederförmiger Ausbildungen wird auf den vorherigen Absatz Bezug genommen.

In einer weiteren Ausführungsform weisen die osteokonduktiven Stützkörper, insbesondere Formkörper, einen eckenlosen, Querschnitt auf. Beispielsweise können die Strukturelemente einen ovalförmigen, insbesondere kreisförmigen oder elliptischen, Querschnitt aufweisen.

In einer weiteren Ausführungsform sind die osteokonduktiven Stützkörper, insbesondere Formkörper, nicht-polyederförmig, insbesondere kugel-, kegel-, kegelstumpf-, ring-, toroid-, oder kreiszylinderförmig, ausgebildet.

Weiterhin können die osteokonduktiven Stützkörper, insbesondere Formkörper, unterschiedlich nicht-polyederförmig ausgebildet sein. Mit anderen Worten können die osteokonduktiven Stützkörper, insbesondere Formkörper, in unterschiedlichen Nicht-Polyederformen vorliegen. Bezüglich möglicher nicht-polyederförmiger Ausbildungen wird auf den vorherigen Absatz Bezug genommen.

In einer weiteren Ausführungsform sind die osteokonduktiven Stützkörper, insbesondere Formkörper, in Form von Oligopoden, d.h. oligopodenförmig, ausgebildet.

Die Oligopoden können konusförmig und insbesondere rotationssymmetrisch ausgebildete Beine aufweisen. Die Beine können einen Konuswinkel von 5° bis 25°, insbesondere 7° bis 15°, aufweisen.

Weiterhin können die Oligopoden Beine mit einer Länge von 0.5 mm bis 5 mm, insbesondere 1.5 mm bis 2.5 mm, aufweisen.

Des Weiteren können die Oligopoden Beine mit einem mittleren Durchmesser von 0.2 mm bis 3 mm, insbesondere 0.3 mm bis 0.7 mm, aufweisen.

Die Oligopoden können ausgewählt sein aus der Gruppe bestehend aus Tripoden, Tetrapoden, Pentapoden, Hexapoden, Heptapoden, Oktapoden und Mischungen aus wenigstens zwei der genannten Oligopoden.

Erfindungsgemäß ist es besonders bevorzugt, wenn die osteokonduktiven Stützkörper tetrapodenförmig ausgebildet sind. Eine tetrapodenförmige Ausgestaltung erlaubt ein besonders wirkungsvolles gegenseitiges Ineinandergreifen der osteokonduktiven Stützkörper.

In einer weiteren Ausführungsform weisen die osteokonduktiven Stützkörper, insbesondere Formkörper, längliche Strukturelemente auf. Insbesondere können die osteokonduktiven Stützkörper, insbesondere Formkörper, aus länglichen Strukturelementen zusammengesetzt sein.

Unter dem Ausdruck "längliche Strukturelemente" sollen im Sinne der vorliegenden Erfindung Strukturelemente mit einem Länge-Breite-Verhältnis oder Länge-Durchmesser-Verhältnis > (gesprochen: größer) 1 verstanden werden.

Bevorzugt weisen die osteokonduktiven Stützkörper, insbesondere Formkörper, längliche und gerade verlaufende Strukturelemente auf. Bevorzugt sind die osteokonduktiven Stützkörper, insbesondere Formkörper, aus länglichen und gerade verlaufenden Strukturelementen zusammengesetzt.

Die länglichen Strukturelemente sind vorzugsweise polyederförmig, insbesondere quaderförmig, würfelförmig, prismenförmig, pyramidenförmig, pyramidenstumpfförmig oder spatförmig, angeordnet. Mit anderen Worten bilden die Strukturelemente eines jeden osteokonduktiven Stützkörpers, insbesondere Formkörpers, vorzugsweise eine polyederförmige, insbesondere quaderförmige, würfelförmige, prismenförmige, pyramidenförmige, pyramidenstumpfförmige oder spatförmige, Anordnung aus.

Die länglichen Strukturelemente können eine Länge von 0.4 mm bis 5 mm, insbesondere 0.8 mm bis 4.5 mm, bevorzugt 1 mm bis 4 mm, aufweisen.

Weiterhin können die länglichen Strukturelemente eine Breite oder einen Durchmesser von 0.4 mm bis 5 mm, insbesondere 0.8 mm bis 4.5 mm, bevorzugt 1 mm bis 4 mm, aufweisen.

Des Weiteren können die länglichen Strukturelemente einen eckenlosen Querschnitt aufweisen. Beispielsweise können die Strukturelemente einen ovalförmigen, insbesondere kreisförmigen oder elliptischen, Querschnitt aufweisen.

Alternativ können die Strukturelemente einen polygonen Querschnitt aufweisen. Beispielsweise können die Strukturelemente einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt aufweisen.

Osteokonduktive Stützkörper, insbesondere in Form von Formkörpern, mit länglichen und insbesondere gerade verlaufenden Strukturelementen, insbesondere wie in den vorhergehenden Ausführungsformen beschrieben, haben den Vorteil, dass durch die gegenseitige Anordnung der Strukturelemente pro Stützkörper, insbesondere Formkörper, zusätzliches Hohlraumvolumen geschaffen werden kann, wodurch sich die osteokonduktiven Eigenschaften der Stützkörper, insbesondere Formkörper, und mithin des Implantats zusätzlich verbessern lassen. Insbesondere können hierdurch die Porengröße (absolutes Hohlraumvolumen) sowie die Porosität (Verhältnis Materialvolumen zu Hohlraumvolumen) von humanem oder tierischem Knochen optimal nachgebildet werden.

In einer weiteren Ausführungsform sind die osteokonduktiven Stützkörper unregelmäßig geformt.

Die osteokonduktiven Stützkörper können insbesondere in partikulärer Form, d.h. in Form von Partikeln, vorliegen.

In einer weiteren Ausführungsform sind die osteokonduktiven Stützkörper als gebrochenes Material ausgebildet.

In einer weiteren Ausführungsform sind die osteokonduktiven Stützkörper als nicht gebrochenes Material ausgebildet. Beispielsweise können die osteokonduktiven Stützkörper als additiv gefertigtes Material, d.h. als Material, welches mittels eines additiven Fertigungsverfahrens hergestellt ist, ausgebildet sein.

Bevorzugt sind die osteokonduktiven Stützkörper als Schüttgut, insbesondere als Granulat, ausgebildet.

Unter dem Ausdruck "Schüttgut" soll im Sinne der vorliegenden Erfindung ein partikuläres Material, d.h. ein Material in Form von Partikeln, verstanden werden, dessen Partikel wenigstens eine Abmessung oder Dimension kleiner als 7 mm, vorzugsweise in einem Größenbereich von 0.5 mm bis 5 mm, aufweisen. Bei der wenigstens einen Abmessung bzw. Dimension kann es sich insbesondere um die Höhe und/oder Länge und/oder Breite (Dicke) und/oder den Durchmesser, insbesondere mittleren Durchmesser, der Partikel handeln. Unter dem Ausdruck "Granulat" soll im Sinne der vorliegenden Erfindung ein partikuläres Material aus unregelmäßig geformtem, insbesondere gebrochenem und/oder gesiebtem, Material verstanden werden.

Vorzugsweise weisen die osteokonduktiven Stützkörper wenigstens eine Abmessung oder Dimension in einem Größenbereich von 0.5 mm bis 5 mm, insbesondere 0.1 mm bis 3 mm, vorzugsweise 1 mm bis 2 mm, auf. Bei der wenigstens einen Abmessung bzw. Dimension kann es sich insbesondere um die Höhe und/oder Breite (Dicke) und/oder Länge und/oder den Durchmesser, insbesondere mittleren Durchmesser, der osteokonduktiven Stützkörper handeln.

In einer weiteren Ausführungsform sind die osteokonduktiven Stützkörper gegeneinander beweglich, insbesondere gegeneinander verschiebbar.

In einer weiteren Ausführungsform sind die osteokonduktiven Stützkörper impaktierbar, d.h. gegenseitig einklemmbar oder gegenseitig verkeilbar, ausgebildet.

In einer weiteren Ausführungsform liegen die osteokonduktiven Stützkörper impaktiert, d.h. gegenseitig eingeklemmt oder gegenseitig verkeilt, vor.

In einer weiteren Ausführungsform sind die osteokonduktiven Stützkörper, bevorzugt mittels Impaktieren, in eine dreidimensionale und insbesondere Hohl- und/oder Zwischenräume aufweisende Struktur oder Matrix überführbar oder liegen in einer solchen Struktur bzw. Matrix vor. Eine solche Struktur bzw. Matrix kann im Sinne der vorliegenden Erfindung auch als osteokonduktive Leitstruktur bzw. osteokonduktive Leitmatrix bezeichnet werden.

Die Hohl- bzw. Zwischenräume der Struktur bzw. Matrix können einen Durchmesser, insbesondere mittleren Durchmesser, von 0.1 mm bis 1.2 mm, insbesondere 0.2 mm bis 1 mm, bevorzugt 0.3 mm bis 0.8 mm, aufweisen.

Weiterhin kann die Struktur bzw. Matrix mit besonderem Vorteil ein Hohl- bzw. Zwischenraumvolumen von 5 % bis 95 %, insbesondere 10 % bis 80 %, bevorzugt 20 % bis 70 %, aufweisen. Ein derartiges Hohl- bzw. Zwischenraumvolumen spiegelt das Porenvolumen einer menschlichen oder tierischen Spongiosa optimal wieder und bewirkt eine Verbesserung der Osteokonduktivität des Implantats sowie insbesondere der biologischen Rekonstruktion eines knöchernen Defekts.

Weiterhin sind die Hohl- bzw. Zwischenräume der Struktur bzw. Matrix vorzugsweise wenigstens zum Teil miteinander verbunden. Auf diese Weise spiegelt die dreidimensionale Struktur die Porosität, insbesondere interkonnektierende Porosität, der menschlichen oder tierischen Spongiosa optimal wieder. Dadurch kann mit besonderem Vorteil ebenfalls ein Einwachsen von Knochengewebe in einen defekten Knochenbereich und insbesondere ein Durchwachsen eines defekten Knochenbereichs mit vitalem Knochengewebe angeregt und/oder verstärkt werden. Dies trägt ebenso zu einer Verbesserung der osteokonduktiven Eigenschaften des Implantats und insbesondere der biologischen Rekonstruktion eines Knochendefekts bei.

Weiterhin weist die Struktur bzw. Matrix vorzugsweise ein Elastizitätsmodul, nachfolgend auch als E-Modul bezeichnet, von 10 MPa bis 10 GPa, insbesondere 50 MPa bis 1 GPa, bevorzugt 80 MPa bis 350 MPa, auf. Unter dem Ausdruck "Elastizitätsmodul (E-Modul)" soll im Sinne der vorliegenden Erfindung das Elastizitätsmodul verstanden werden. Der Betrag des Elastizitätsmoduls ist umso größer, je mehr Widerstand ein Material seiner elastischen Verformung entgegensetzt. Ein Körper aus einem Material mit einem hohen Elastizitätsmodul ist daher steifer als ein Körper gleicher Ausgestaltung (gleiche geometrische Abmessung), welcher aus einem Material mit einem niedrigen Elastizitätsmodul besteht. Die in diesem Absatz offenbarten Werte für das Elastizitätsmodul spiegeln in optimaler Weise die entsprechenden Werte von spongiösem Knochen wieder, welcher ein Elastizitätsmodul von 100 MPa bis 1.000 MPa aufweist.

Aufgrund der im vorherigen Absatz beschriebenen geringen E-Module können die osteokonduktiven Stützkörper gleichmäßig, d.h. homogen, mechanisch belastet werden. Insbesondere können auch die Hohl- bzw. Zwischenhohlräume der in den vorherigen Absätzen beschriebenen Struktur bzw. Matrix mechanisch belastet werden. Durch eine gleichmäßige bzw. homogene mechanische Belastung der osteokonduktiven Stützkörper und mithin des Implantats ist wiederum mit besonderem Vorteil eine Knochenbildung, insbesondere Knochenneubildung, innerhalb eines gesamten knöchernen Defektbereichs erzielbar.

In einer weiteren unter Stütz- bzw. Tragfähigkeitsgesichtspunkten vorteilhaften Ausführungsform weisen die osteokonduktiven Stützkörper Öffnungen oder Vertiefungen, insbesondere durchgehende Öffnungen, auf. Die Öffnungen bzw. Vertiefungen können ausgewählt sein aus der Gruppe bestehend aus Löchern, Poren, Rissen, Schlitzen, Ritzen, Spalten, Kerben und Kombinationen aus wenigstens zwei der genannten Öffnungen bzw. Vertiefungen.

Eine solche Ausgestaltung der Stützkörper hat den Vorteil, dass sich die Stützkörper bei Belastung (leichter) komprimieren, insbesondere deformieren, lassen. Entsprechende zu einer Stützkörperkomprimierung führende Belastungen können beispielsweise bei einer Kraftbeaufschlagung durch einen Anwender, vorzugsweise einen Chirurgen, auftreten. Dadurch lässt sich eine Verdichtung, insbesondere Impaktierung, der osteokonduktiven Stützkörper zusätzlich verbessern, was wiederum verbesserte Tragfähigkeitseigenschaften des Implantats zur Folge hat.

Bei den Öffnungen bzw. Vertiefungen kann es sich weiterhin um geometrisch definierte oder undefinierte Öffnungen bzw. Vertiefungen handeln.

Insbesondere können die Öffnungen bzw. Vertiefungen einen ovalen, insbesondere kreisförmigen oder elliptischen, Querschnitt aufweisen. Alternativ oder in Kombination können die Öffnungen bzw. Vertiefungen einen polygonen, insbesondere dreieckförmigen, quadratförmigen, rechteckförmigen, fünfeckförmigen, sechseckförmigen, siebeneckförmigen, achteckförmigen, neuneckförmigen, zehneckförmigen oder sternenförmigen, Querschnitt aufweisen.

Die Öffnungen bzw. Vertiefungen können einen Durchmesser von 0.01 mm bis 5 mm, insbesondere 0.1 mm bis 4 mm, bevorzugt 0.5 mm bis 3 mm, aufweisen. Derartige Durchmesser können bevorzugt sein, wenn die Öffnungen als durchgehende Öffnungen ausgebildet sind, durch welche, wie nach folgend noch näher erläutert werden wird, ein Zugelement zum Zwecke des miteinander Verbindens oder Verzurrens der osteokonduktiven Stützkörper hindurch geführt werden soll.

In einer alternativen Ausführungsform weisen die Öffnungen bzw. Vertiefungen einen Durchmesser, insbesondere mittleren Durchmesser, von 60 µm bis 500 µm, bevorzugt 100 µm bis 400 µm, auf. Derartige Durchmesser sind bevorzugt, wenn die Öffnungen bzw. Vertiefungen als Poren ausgestaltet sind.

Bevorzugt handelt es sich bei den Öffnungen bzw. Vertiefungen um Poren. Anders ausgedrückt, sind die osteokonduktiven Stützkörper vorzugsweise offenporig ausgebildet. Insbesondere können die osteokonduktiven Stützkörper eine interkonnektierende Porosität aufweisen.

In einer weiteren Ausführungsform weisen die osteokonduktiven Stützkörper Fasern auf. Bei den Fasern kann es sich grundsätzlich um Kurz- und/oder Langfasern handeln.

Unter dem Ausdruck "Kurzfasern" sollen im Sinne der vorliegenden Erfindung Fasern mit einer Länge von 0.01 mm bis 1 mm, insbesondere 0.1 mm bis 1 mm, bevorzugt 0.5 mm bis 1 mm, verstanden werden.

Unter dem Ausdruck "Langfasern" sollen im Sinne der vorliegenden Erfindung Fasern mit einer Länge > (gesprochen: größer) 1 mm verstanden werden.

Bei den Kurz- bzw. Langfasern kann es sich um Metallfasern und/oder Polymerfasern handeln. Bezüglich möglicher Metalle und/oder Polymere, aus welchen die Kurz- bzw. Langfasern gebildet sein können, wird auf die noch im Folgenden im Zusammenhang der Einbringhilfe genannten Metalle bzw. Polymere Bezug genommen.

In einer weiteren Ausführungsform weist das Implantat ferner ein Zugelement auf. Das Zugelement ist vorzugsweise dazu ausgebildet, durch durchgehende Öffnungen der osteokonduktiven Stützkörper hindurch geführt zu werden. Dadurch ist es mit besonderem Vorteil möglich die osteokonduktiven Stützkörper miteinander zu verbinden oder miteinander zu verzurren. Das Zugelement ist daher zweckmäßigerweise ein längliches Zugelement.

Vorzugsweise handelt es sich bei dem Zugelement um ein textiles, insbesondere fadenförmiges, Zugelement. Beispielsweise kann es sich bei dem Zugelement um einen Faden (Zugfaden), insbesondere um einen monofilen, pseudomonofilen oder multifilen Faden, handeln. Insbesondere kann es sich bei dem Zugelement um ein chirurgisches Nahtmaterial handeln.

Weiterhin kann es sich bei dem Zugelement um ein textiles Flächengebilde, insbesondere in Form eines Gewirks, Geflechts, Gestricks, Geleges, Vlies oder Vliesstoffs, handeln. Bevorzugt ist das Zugelement ein Netz, insbesondere ein kleinporiges Netz, vorzugsweise ein Herniennetz. Durch ein Einbinden der osteokonduktiven Stützkörper in ein netzförmiges Zugelement kann eine regelmäßige Anordnung der Stützkörper erzielt werden.

Alternativ kann es sich bei dem Zugelement um einen Draht (Zugdraht) handeln.

Weitere Vorteile, welche bei Verwendung des Zugelements realisiert werden können, werden nachfolgend beschrieben.

Die Verwendung eines Zugelements ermöglicht ein Befestigen oder Verzurren der osteokonduktiven Stützkörper, wodurch eine sofortige Festigkeitserhöhung der osteokonduktiven Stützkörper untereinander und damit des Implantats erreicht werden kann. Dies kann mit besonderem Vorteil dazu führen, dass eine geringere Menge des Knochenzements erforderlich ist, um ein stütz- bzw. tragfähiges Implantat zu erhalten. Außerdem lässt sich durch eine derartige Festigkeitserhöhung der osteokonduktiven Stützkörper das Risiko senken, dass eine durch die Stützkörper gebildete Gerüststruktur nach einem Sprödbruch auseinanderbricht. Weiterhin kann durch ein Befestigen bzw. Verzurren der osteokonduktiven Stützkörper mit besonderem Vorteil eine offenporige Gerüststruktur realisiert werden. Weiterhin besteht die Möglichkeit, dass eine Zugelement-Stützkörper-Einheit (oder gegebenenfalls mehrere Zugelement-Stützkörper-Einheiten) an ein weiteres Implantat und/oder an einen Knochen fixiert und dadurch ortsstabil befestigt werden kann (können). Die Zugelement-Stützkörper-Einheit (oder Zugelement-Stützkörper-Einheiten) kann (können) durch die Befestigung an ein weiteres Implantat beispielsweise an einen angefrischten Knochen angedrückt werden. Hierdurch ist eine optimale Anbindung an den Knochen möglich, und der resultierende Druck auf den Knochen fördert das Knochenwachstum. Die Kraftübertragung am Knochendefekt übernimmt bevorzugt das Implantat. Dadurch entfällt der Druckreiz, welcher den Knochen zum Knochenaufbau anregt (stress shielding). Dieser Druckreiz kann durch das bzw. die unter Druck zum Knochen stehenden Zugelement-Stützkörper-Einheiten aufgebaut werden.

Das Zugelement kann ein Material, insbesondere Polymer und/oder Metall, aufweisen oder aus einem Material, insbesondere Polymer und/oder Metall, bestehen, wie es nachfolgend noch im Zusammenhang der Einbringhilfe näher beschrieben werden wird.

In einer weiteren Ausführungsform sind die osteokonduktiven Stützkörper derart ausgestaltet, dass sie form-, kraft- und/oder stoffschlüssig miteinander verbindbar sind. Bevorzugt sind die osteokonduktiven Stützkörper derart ausgestaltet, dass sie formschlüssig miteinander verbindbar sind. Beispielsweise können die Stützkörper derart ausgestaltet sein, dass sie über ein Stecksystem bzw. nach Art eines Stecksystems miteinander verbunden werden können. Das Stecksystem kann dabei auf einem sogenannten Zapfen-Loch-Prinzip beruhen, bevorzugt mit einem Hinterschnitt zur besseren Verankerung der osteokonduktiven Stützkörper. Hierzu können ein Teil der osteokonduktiven Stützkörper mit Zapfen und ein anderer Teil der osteokonduktiven Stützkörper mit passenden Zapflöchern oder Schlitzen versehen sein.

In einer weiteren Ausführungsform sind die osteokonduktiven Stützkörper form-, kraft- und/oder stoffschlüssig miteinander verbunden. Bevorzugt sind die Stützkörper formschlüssig miteinander verbunden. Beispielsweise können die osteokonduktiven Stützkörper über ein Stecksystem bzw. nach Art eines Stecksystems miteinander verbunden sein. Bezüglich des Stecksystems wird auf den vorherigen Absatz Bezug genommen.

In einer weiteren Ausführungsform sind die Stützkörper derart ausgestaltet, dass sie form-, kraft- und/oder stoffschlüssig mit einem anderen Implantat verbindbar sind. Bevorzugt sind die Stützkörper derart ausgestaltet, dass sie formschlüssig mit einem anderen Implantat verbindbar sind. Beispielsweise können die Stützkörper derart ausgestaltet sein, dass sie über ein Stecksystem bzw. nach Art eines Stecksystems mit einem Implantat verbunden werden können. Das Stecksystem kann dabei auf einem sogenannten Zapfen-Loch-Prinzip beruhen.

Hierzu können die Stützkörper mit einem Zapfen versehen sein, und das andere Implantat kann komplementäre Zapfenlöcher oder Schlitze aufweisen. Die umgekehrten Verhältnisse können erfindungsgemäß ebenso möglich sein.

In einer weiteren Ausführungsform sind die osteokonduktiven Stützkörper über längliche Verbindungselemente miteinander verbunden. Bevorzugt ragen die Verbindungselemente hierzu in Ausnehmungen bzw. Öffnungen der Stützkörper. Bezüglich möglicher Ausgestaltungen der Ausnehmungen bzw. Öffnungen der Stützkörper wird auf die vorangegangenen Ausführungen Bezug genommen. Die Verbindungselemente sowie die osteokonduktiven Stützkörper können ein gleiches Material aufweisen oder aus einem gleichen Material bestehen. Bevorzugt ist es jedoch, wenn die Verbindungselemente und die osteokonduktiven Stützkörper verschiedene Materialien aufweisen oder aus verschiedenen Materialien bestehen.

Die länglichen Verbindungselemente können ein Material, insbesondere Polymer und/oder Metall, aufweisen oder aus einem Material, insbesondere Polymer und/oder Metall, bestehen, wie es nachfolgend noch im Zusammenhang der Einbringhilfe näher beschrieben werden wird.

In einer weiteren Ausführungsform weisen die osteokonduktiven Stützkörper einen Anteil von 10 Gew.-% bis 95 Gew.-%, insbesondere 20 Gew.-% bis 90 Gew.-%, bevorzugt 30 Gew.-% bis 70 Gew.-%, auf, bezogen auf das Gesamtgewicht des Implantats.

In einer weiteren Ausführungsform ist die Einbringhilfe wenigstens teilweise flexibel oder elastisch ausgebildet. Eine wenigstens teilweise flexibel bzw. elastisch ausgebildete Einbringhilfe hat den Vorteil, dass die Einbringhilfe, beispielsweise durch Biegen, leichter an die Kontur eines Knochens angepasst werden kann. Ein weiterer Vorteil besteht darin, dass eine wenigstens teilweise flexibel bzw. elastisch ausgebildete Einbringhilfe eine Druck- oder Kraftbeaufschlagung der osteokonduktiven Stützkörper nach Einbringen in einen knöchernen Defekt erleichtert. Dies wiederum begünstigt ein Verdichten, insbesondere Impaktieren, der osteokonduktiven Stützkörper, vorzugsweise unter Ausbildung einer osteokonduktiven Leitstruktur, und mithin die Erzielung einer ausreichenden Sekundärstabilität und insbesondere biologischen Rekonstruktion eines zu behandelnden Knochendefekts.

Die Einbringhilfe kann insbesondere vollständig flexibel bzw. elastisch ausgebildet sein.

In einer alternativen Ausführungsform ist die Einbringhilfe starr ausgebildet.

In einer weiteren Ausführungsform ist die Einbringhilfe forminstabil ausgebildet. Eine forminstabile Einbringhilfe hat den Vorteil, dass sie die Anpassung des Implantats an einen zu behandelnden Knochendefekt erleichtert. Eine bevorzugte forminstabile Ausgestaltung der Einbringhilfe stellt insbesondere ein Netz oder Bindemittel dar, worauf im Folgenden noch näher eingegangen werden wird.

In einer alternativen Ausführungsform ist die Einbringhilfe formstabil ausgebildet. Eine bevorzugte formstabile Ausgestaltung der Einbringhilfe stellt insbesondere eine plattenförmige Abdeckung dar, worauf im Folgenden ebenfalls noch näher eingegangen werden wird.

In einer weiteren Ausführungsform weist die Einbringhilfe ein Material auf oder besteht aus einem Material, welches ausgewählt ist aus der Gruppe bestehend aus Protein wie extrazelluläres Protein, Polysaccharid wie Mucopolysaccharid und/oder Cellulosederivat, biologisches Gewebe, aufbereitetes oder gereinigtes biologisches Gewebe, extrazelluläre Matrix, Polycarbonat wie Polytrimethylencarbonat, Poly-para-dioxanon, Polyhydroxyalkanoat, Polyvinylalkohol, Polyethylenglykole, Ethylenoxid-Propylenoxid-Copolymere (EO-PO-Copolymere), Ethylenoxid-Propylenoxid-Blockcopolymere (EO-PO-Blockcopolymere), Glycerin, Polyolefin, Polyester, Polyamid, Polyurethan, Polyacrylsäuren, Acrylsäure-Homopolymere, Acrylsäure-Copolymere, Polyvinylpyrrolidon-Homopolymere, Polyvinylpyrrolidon-Copolymere, Elastomer wie thermoplastisches Elastomer, Polyetherketon, organisches Polysulfid, Metall, Legierung und Kombinationen, insbesondere Mischungen oder Verbundstrukturen, aus wenigsten zwei der genannten Materialien.

Das Protein kann insbesondere ausgewählt sein aus der Gruppe bestehend aus Collagen, Gelatine, Elastin, Retikulin, Fibronektin, Fibrin, Laminin, Albumin und Mischungen aus wenigstens zwei der genannten Proteine. Bei dem Collagen handelt es sich vorzugsweise um Collagen Typ I, Collagen Typ III oder um eine Mischung enthaltend oder bestehend aus Collagen Typ I und Collagen Typ III.

Das Polysaccharid kann insbesondere ausgewählt sein aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Carboxyethylcellulose, Algininsäure, Alginate, Chitin, Chitosan, Hyaluronsäure, Dextransulfat, Heparin, Heparansulfat, Chondroitinsulfat wie Chondroitin-4-sulfat und/oder Chondroitin-6-sulfat, Dermatansulfat, Keratansulfat und Mischungen aus wenigstens zwei der genannten Polysaccharide.

Bei dem biologischen Gewebe kann es sich insbesondere um ein tierisches oder xenogenes, bevorzugt bovines, equines oder porcines, Gewebe handeln.

Das Gewebe kann grundsätzlich ausgewählt sein aus der Gruppe bestehend aus Perikard, Peritoneum, Dünndarm-Submukosa, Magen-Submukosa, Harnblasen-Submukosa, Uterus-Submukosa, Serosa und Mischungen aus wenigstens zwei der genannten Gewebe.

Bevorzugt ist das Gewebe ausgewählt aus der Gruppe bestehend aus Perikard (Herzbeutel), Pericardium fibrosum, Pericardium serosum, Epikard, Plattenepithel, Tunica serosa, Muskel wie beispielsweise Myokard und Mischungen aus wenigstens zwei der genannten Gewebe.

Besonders bevorzugt handelt es sich bei dem Gewebe um Perikard, insbesondere um bovines Perikard, d.h. um Rinderperikard.

Bei dem aufbereiteten bzw. gereinigten biologischen Gewebe kann es sich insbesondere um ein von nichtcollagenen Bestandteilen, bevorzugt Fetten und/oder Enzymen und/oder nichtcollagenen Proteinen, befreites biologisches Gewebe handeln. Besonders bevorzugt handelt es sich bei dem aufbereiteten bzw. gereinigten Gewebe um ein aus Rinderperikard hergestelltes und von nichtcollagenen Bestandteilen, insbesondere Fetten, Enzymen und nichtcollagenen Proteinen, gereinigtes und lyophilisiertes Collagenmaterial. Ein derartiges Material wird von der Anmelderin unter der Bezeichnung Lyoplant^{®} bereits kommerziell für den Duraersatz angeboten. Bezüglich weiterer Merkmale des Gewebes wird auf die vorangegangenen Ausführungen Bezug genommen.

Bei der extrazellulären Matrix kann es sich insbesondere um eine extrazelluläre Matrix eines biologischen Gewebes handeln. Bevorzugt handelt es sich bei der extrazellulären Matrix um die extrazelluläre Matrix eines tierischen, insbesondere bovinen, equinen oder procinen, Gewebes. Bezüglich weiterer Merkmale des Gewebes wird ebenfalls auf die vorangegangenen Ausführungen Bezug genommen.

Das Polyhydroxyalkanoat kann insbesondere ausgewählt sein aus der Gruppe bestehend aus Polyglycolid, Polylactid, Polycaprolacton, Poly-3-hydroxybutyrat, Poly-4-hydroxybutyrat, Copolymere aus wenigstens zwei der genannten Polymere und Mischungen (Blends) aus wenigstens zwei der genannten Polymere.

Das Polyolefin kann insbesondere ausgewählt sein aus der Gruppe bestehend aus Polyethylen (PE), Polyethylen niedriger Dichte, Polyethylen hoher Dichte, hochmolekulares Polyethylen (HMWPE), ultrahochmolekulares Polyethylen (UHMWPE), Polypropylen (PP), Polytetrafluorethylen (PTFE), Polyvinylidenfluorid, Polyvinylchlorid, Polytetrafluorpropylen, Polyhexafluorpropylen, Copolymere aus wenigstens zwei der genannten Polyolefine und Mischungen (Blends) aus wenigstens zwei der genannten Polyolefine.

Der Polyester kann insbesondere ausgewählt sein aus der Gruppe bestehend aus Polyethylenterephthalat, Polypropylenterephthalat, Polybutylenterephthalat, Copolymere aus wenigstens zwei der genannten Polyester und Mischungen (Blends) aus wenigstens zwei der genannten Polyester.

Das Polyamid kann insbesondere ausgewählt sein aus der Gruppe bestehend aus Polyamid 6 (Polymer aus ε-Caprolactam- oder ω-Aminocapronsäure-Einheiten), Polyamid 66 (Polymer aus Hexamethylendiamin- und Adipinsäureeinheiten), Polyamid 69 (Polymer aus Hexamethylendiamin- und Azelainsäureeinheiten), Polyamid 612 (Polymer aus Hexamethylendiamin- und Dodecandisäureeinheiten), Polyamid 11 (Polymer aus 11-Aminoundecansäureeinheiten), Polyamid 12 (Polymer aus Laurinlactam- oder ω-Aminododecansäureeinheiten), Polyamid 46 (Polymer aus Tetramethylendiamin- und Adipinsäureeinheiten), Polyamid 1212 (Polymer aus Dodecandiamin- und Dodecandisäureeinheiten), Polyamid 6/12 (Polymer aus Caprolactam- und Laurinlactameinheiten), Polyamid 66/610 (Polymer aus Hexamethylendiamin-, Adipinsäure- und Sebacinsäureeinheiten), Copolymere aus wenigstens zwei der genannten Polyamide und Mischungen (Blends) aus wenigstens zwei der genannten Polyamide.

Das thermoplastische Elastomer kann insbesondere ausgewählt sein aus der Gruppe bestehend aus thermoplastisches Copolyamid, thermoplastisches Polyesterelastomer, thermoplastischer Copolyester, thermoplastisches Elastomer auf Olefinbasis, Styrol-Blockcopolymer, thermoplastisches Elastomer auf Urethanbasis, vernetztes thermoplastisches Elastomer auf Olefinbasis, Copolymere aus wenigstens zwei der genannten Elastomere und Mischungen (Blends) aus wenigstens zwei der genannten Elastomere.

Das Polyetherketon kann insbesondere ausgewählt sein aus der Gruppe bestehend aus Polyetherketonketon, Polyetheretheretherketon, Polyetheretherketonketon, Polyetherketonetherketonketon, Copolymere aus wenigstens zwei der genannten Polyetherketone und Mischungen (Blends) aus wenigstens zwei der genannten Polyetherketone.

Das Metall kann insbesondere ausgewählt sein aus der Gruppe bestehend aus Titan und Tantal.

Die Legierung kann insbesondere ausgewählt sein aus der Gruppe bestehend aus Stahl wie Edelstahl, rostfreier Stahl oder hochlegierte Stähle, insbesondere mit Chrom, Nickel, Duplexstähle, und Mischungen davon.

Die Einbringhilfe ist als Kombination, insbesondere Verbund, aus einem textilen Flächengebilde, vorzugsweise Netz, und einem Bindemittel ausgebildet.

Bevorzugt sind die osteokonduktiven Stützkörper durch das Bindemittel an dem textilen Flächengebilde, vorzugsweise Netz, fixiert bzw. mit dem textilen Flächengebilde, vorzugsweise Netz, verklebt. Besonders bevorzugt sind die osteokonduktiven Stützkörper durch das Bindemittel nur auf einer Seite, insbesondere nur auf einer im eingebrachten Zustand knochendefektzugewandten Seite, des textilen Flächengebildes, vorzugsweise Netzes, an diesem fixiert bzw. mit diesem verklebt. Bezüglich weiterer Merkmale und Vorteile des textilen Flächengebildes, insbesondere Netzes, sowie des Bindemittels wird vollständig auf die bisherige Beschreibung Bezug genommen.

Das textile Flächengebilde kann insbesondere ausgewählt sein aus der Gruppe bestehend aus Gewebe, Gewirke, Gestrick, Geflecht, Vlies, Vliesstoff, Netz, Filz und Maschenstoff.

Weiterhin kann das textile Flächengebilde Fäden aufweisen oder aus Fäden gebildet sein, welche ausgewählt sind aus der Gruppe bestehend aus Monofilamente, Pseudomonofilamente, Multifilamente und Kombinationen aus wenigstens zwei der genannten Fäden.

Gemäß einer besonders bevorzugten Ausführungsform handelt es sich bei dem textilen Flächengebilde um ein Netz, insbesondere gewirktes Netz. Mit anderen Worten ist es besonders bevorzugt, wenn die Einbringhilfe ein Netz, insbesondere gewirktes Netz, aufweist oder als Netz, insbesondere gewirktes Netz, ausgebildet ist.

In einer weiteren Ausführungsform weist das textile Flächengebilde, insbesondere Fäden davon, einen Zusatzstoff, wie beispielsweise einen Wirkstoff und/oder ein Röntgenkontrastmittel, auf. Bezüglich geeigneter Zusatzstoffe sei auf die im Folgenden noch näher erläuterten Zusatzstoffe verwiesen.

Grundsätzlich können Fäden des textilen Flächengebildes eine gleiche Fadenstärke (Fadendurchmesser) besitzen. Unter Formstabilitätsgesichtspunkten kann es jedoch bevorzugt sein, wenn das textile Flächengebilde Fäden mit unterschiedlichen Fadenstärken (Fadendurchmessern) aufweist.

Weiterhin können Fäden des textilen Flächengebildes, insbesondere wenigstens teilweise, gefärbt, beispielsweise weiß und/oder blau gefärbt, vorliegen. Bei den gefärbten Fäden kann es sich insbesondere um Orientierungsfäden handeln, welche einem Anwender, bevorzugt einem Chirurgen, die richtige Platzierung der Einbringhilfe und insbesondere des Implantats erleichtern.

In einer weiteren Ausführungsform weist das textile Flächengebilde eine Befestigungseinrichtung auf. Die Befestigungseinrichtung ist vorzugsweise dazu ausgebildet, eine Befestigung des textilen Flächengebildes an oder in einem Knochen zu bewirken.

Die Befestigungseinrichtung kann beispielsweise als Loch, verstärktes Loch, Öse, Hülse, Schlitz, Spalte oder Schlinge ausgebildet sein.

Die Befestigungseinrichtung kann weiterhin textil, insbesondere als Faden oder Fadenschlinge, ausgestaltet sein.

Mit besonderem Vorteil kann die Befestigungseinrichtung aus einem steiferen Material gebildet sein als das textile Flächengebilde.

In einer weiteren Ausführungsform handelt es sich bei dem textilen Flächengebilde, insbesondere Netz, um das von der Anmelderin unter der Bezeichnung Optilene^{®} Mesh erhältliche Produkt. Hierbei handelt es sich um ein gewirktes Netz mit monofilen Polypropylenfäden, einem Flächengewicht von 60 g/m² und einer Porengröße von ca. 1.5 mm.

In einer weiteren Ausführungsform handelt es sich bei dem textilen Flächengebilde, insbesondere Netz, um das von der Anmelderin unter der Bezeichnung Optilene^{®} Mesh LP vertriebene Produkt. Hierbei handelt es sich um ein gewirktes Netz mit monofilen Polypropylenfäden, einem Flächengewicht von 36 g/m² und einer Porengröße von ca. 1.0 mm.

In einer weiteren Ausführungsform handelt es sich bei dem textilen Flächengebilde, insbesondere Netz, um das von der Anmelderin unter der Bezeichnung Optilene^{®} Mesh Elastic vertriebene Produkt. Hierbei handelt es sich um ein gewirktes Netz mit monofilen Polypropylenfäden, einem Flächengewicht von ca. 48 g/m² sowie einer Porengröße von ca. 3.6 mm x 2.8 mm.

Bezüglich weiterer geeigneter Materialien für das textile Flächengebilde, insbesondere Netz, wird auf die bereits im Zusammenhang der Einbringhilfe beschriebenen Materialien, insbesondere Polymere, Bezug genommen.

Die Verwendung eines Bindemittels hat den Vorteil, dass durch Verklebung eine unkontrollierte Verteilung der osteokonduktiven Stützkörper beim Einbringen in einen zu behandelnden Knochendefekt vermieden werden kann.

Die osteokonduktiven Stützkörper sind durch das Bindemittel miteinander verklebt unter Ausbildung einer knetbaren oder pastösen Masse oder Zubereitung. Eine knetbare bzw. pastöse Beschaffenheit einer durch das Bindemittel und die osteokonduktiven Stützkörper erzeugten Masse bzw. Zubereitung erlaubt mit besonderem Vorteil eine intraoperative Änderung von deren Form und/oder Menge. Dies wiederum erleichtert die Anpassung des Implantats an einen zu behandelnden Knochendefekt und verbessert mithin die Handhabung des Implantats.

In einer weiteren Ausführungsform weist das Bindemittel ein Protein wie extrazelluläres Protein, auf oder besteht aus einem Protein wie extrazelluläres Protein. Das Protein ist vorzugsweise ausgewählt aus der Gruppe bestehend aus Collagen, Gelatine, Elastin, Laminin, Retikulin, Fibronektin, Fibrin, Albumin und Mischungen aus wenigstens zwei der genannten Proteine. Bezüglich weiterer geeigneter Proteine wird auf die bereits im Zusammenhang der Einbringhilfe beschriebenen Proteine Bezug genommen.

In einer weiteren Ausführungsform weist das Bindemittel ein Polysaccharid wie Cellulosederivat und/oder Mucopolysaccharid auf. Das Polysaccharid ist vorzugsweise ausgewählt aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Carboxyethylcellulose, Algininsäure, Alginate, Chitin, Chitosan, Hyaluronsäure, Dextransulfat, Heparin, Heparansulfat, Chondroitinsulfat wie Chondroitin-4-sulfat und/oder Chondroitin-6-sulfat, Dermatansulfat, Keratansulfat und Mischungen aus wenigstens zwei der genannten Polysaccharide.

In einer weiteren Ausführungsform weist das Bindemittel ein synthetisches Polymer auf. Das synthetische Polymer ist vorzugsweise ausgewählt aus der Gruppe bestehend aus Polyvinylalkohol, Polyethylenglykole, Ethylenoxid-Propylenoxid-Copolymere (EO-PO-Copolymere), Ethylenoxid-Propylenoxid-Blockcopolymere (EO-PO-Blockcopolymere), Acrylsäure-Homopolymere, Acrylsäure-Copolymere, Polyvinylpyrrolidon-Homopolymere, Polyvinylpyrrolidon-Copolymere und Mischungen davon.

In einer weiteren Ausführungsform weist das Bindemittel eine Mischung aus einem Protein und einem Polysaccharid, eine Mischung aus einem Protein und einem synthetischen Polymer, eine Mischung aus einem Polysaccharid und einem synthetischen Polymer oder eine Mischung aus einem Protein, einem Polysaccharid und einem synthetischen Polymer auf. Bezüglich geeigneter Proteine, geeigneter Polysaccharide sowie geeigneter synthetischer Polymere wird auf die vorangegangenen Absätze Bezug genommen.

Das Bindemittel weist ein Verdünnungsmittel auf. Bei dem Verdünnungsmittel handelt es sich vorzugsweise um Glycerin. Ein verdünnungsmittelhaltiges Bindemittel hat den Vorteil, dass eine vorzugsweise pastöse oder knetbare Konsistenz des Bindemittels bis zum Abschluss einer Knochendefektbehandlung aufrechterhalten werden kann. Dies erleichtert eine Anpassung des Implantats an die Form eines zu behandelnden Knochendefekts.

In einer weiteren Ausführungsform weist das Bindemittel einen Anteil an flüssigem Verdünnungsmittel, vorzugsweise Glycerin und/oder Wasser, von 50 Gew.-% bis 95 Gew.-%, insbesondere 60 Gew.-% bis 90 Gew.-%, bevorzugt 70 Gew.-% bis 80 Gew.-%, auf, bezogen auf das Gesamtgewicht des Bindemittels. Die in diesem Absatz genannten Verdünnungsmittelanteile sind besonders vorteilhaft im Hinblick auf die Aufrechterhaltung einer vorzugsweise pastösen oder knetbaren Konsistenz des Bindemittels bis zum Abschluss einer Knochendefektbehandlung.

In einer weiteren Ausführungsform handelt es sich bei dem Bindemittel um ein wasserfreies Bindemittel oder um ein im Wesentlichen wasserfreies Bindemittel. Unter dem Ausdruck "im Wesentlichen wasserfreies Bindemittel" soll im Sinne der vorliegenden Erfindung ein Bindemittel verstanden werden, welches einen Wassergehalt kleiner 5 Gew.-%, insbesondere kleiner 3 Gew.-%, bevorzugt kleiner 1 Gew.-%, aufweist, bezogen auf das Gesamtgewicht des Bindemittels. Vorzugsweise handelt es sich bei dem Bindemittel um ein wasserfreies Bindemittel oder ein im Wesentlichen wasserfreies Bindemittel, welches Carboxymethylcellulose und Glycerin aufweist. Dadurch kann eine knetartige Konsistenz erreicht werden, welche haftende Eigenschaften aufweist und dadurch gut in einem zu behandelnden Knochendefekt vorfixiert werden kann.

In einer weiteren Ausführungsform weist das Bindemittel eine Auflösungszeit in einem menschlichen oder tierischen (nicht menschlichen) Körper von 30 min bis 144 Stunden, insbesondere 30 min bis 72 Stunden, vorzugsweise 30 min bis 24 Stunden, auf. Dadurch ist mit besonderem Vorteil ein schnelles Einwachsen von Knochengewebe in einen Knochendefekt erzielbar.

In einer weiteren Ausführungsform weist das Implantat ferner eine Befestigungseinrichtung auf. Die Befestigungseinrichtung ist vorzugsweise dazu ausgebildet, eine Befestigung des Implantats an oder in einem Knochen zu bewirken.

Die Befestigungseinrichtung kann beispielsweise als Loch, verstärktes Loch, Öse, Hülse, Schlitz, Spalte oder Schlinge ausgebildet sein.

Die Befestigungseinrichtung kann weiterhin textil, insbesondere als Faden oder Fadenschlinge, ausgestaltet sein.

Mit besonderem Vorteil kann die Befestigungseinrichtung aus einem steiferen Material gebildet sein als das textile Flächengebilde.

In einer weiteren Ausführungsform weist das Implantat ferner eine Armierungsstruktur (Bewehrungsstruktur) auf. Die Armierungsstruktur ist vorzugsweise dazu ausgebildet, eine Armierung, d.h. eine Verstärkung oder Bewehrung, des Implantats zu bewirken.

Die Armierungsstruktur kann beispielsweise eine textile Struktur aufweisen oder aus einer solchen Struktur aufgebaut sein.

Bevorzugt ist die Armierungsstruktur netzförmig, insbesondere in Form eines gewirkten Netzes, ausgebildet. Bei der Armierungsstruktur kann es sich insbesondere um ein Polypropylen-Netz, d.h. ein Netz mit Polypropylenfäden, insbesondere monofilen Polypropylenfäden, handeln. Bevorzugt ist ein gewirktes Polypropylennetz. Beispielsweise kann es sich bei dem Netz um eines der von der Anmelderin unter den Bezeichnungen Optilene^{®} Mesh, Optilene^{®} Mesh LP und Optilene^{®} Mesh Elastic kommerziell vertriebenen Netze handeln.

In einer alternativen Ausführungsform handelt es sich bei der Armierungsstruktur um eine Gitterstruktur, insbesondere um ein Metallgitter. Zum Beispiel kann es sich bei der Armierungsstruktur um ein Titan- oder Tantalgitter handeln.

Bezüglich weiterer geeigneter Materialien für die Armierungsstruktur wird vollständig auf die im Zusammenhang der Einbringhilfe genannten Materialien Bezug genommen.

In einer weiteren Ausführungsform weist das Implantat, insbesondere die osteokonduktiven Stützkörper und/oder die Einbringhilfe, ferner einen Zusatzstoff auf.

Der Zusatzstoff kann das Implantat, insbesondere die osteokonduktiven Stützkörper und/oder die Einbringhilfe, wenigstens teilweise, insbesondere nur teilweise oder vollständig, beschichten. Alternativ kann eine den Zusatzstoff enthaltende Beschichtung das Implantat, insbesondere die osteokonduktiven Stützkörper und/oder die Einbringhilfe, wenigstens teilweise, insbesondere nur teilweise oder vollständig, beschichten.

Bevorzugt liegt der Zusatzstoff zusammen mit den osteokonduktiven Stützkörpern als Mischung, insbesondere heterogene Mischung, vor.

Weiter bevorzugt ist der Zusatzstoff zwischen den osteokonduktiven Stützkörpern angeordnet.

Besonders bevorzugt sind zwischen den osteokonduktiven Stützkörpern ausgebildete oder vorhandene Zwischenräume wenigstens teilweise, insbesondere nur teilweise oder vollständig, mit dem Zusatzstoff gefüllt. In dieser Ausführungsform kann der Zusatzstoff auch als Füllstoff bezeichnet werden.

In einer weiteren Ausführungsform liegt der Zusatzstoff in partikulärer Form, d.h. in Form von Partikeln, insbesondere in Form eines Schüttguts, vorzugsweise Granulats, vor.

In einer weiteren Ausführungsform weist der Zusatzstoff Partikel auf oder liegt in Form von Partikeln vor, welche wenigstens eine Abmessung oder Dimension in einem Bereich von 0.1 mm bis 4 mm, insbesondere 0.5 mm bis 2 mm, vorzugsweise 1 mm bis 1.5 mm, aufweisen. Bei der wenigstens einen Abmessung kann es sich insbesondere um die Höhe und/oder die Breite (Dicke) und/oder die Länge und/oder den Durchmesser, insbesondere mittleren Durchmesser, der Partikel handeln.

In einer weiteren Ausführungsform weist der Zusatzstoff polyederförmige Partikel auf oder liegt in Form von polyederförmigen Partikeln vor. Bezüglich möglicher polyederförmiger Ausbildungen der Partikel wird auf die im Zusammenhang der osteokonduktiven Stützkörper beschriebenen Polyederformen Bezug genommen.

In einer weiteren Ausführungsform weist der Zusatzstoff nicht-polyederförmige Partikel auf oder liegt in Form von nicht-polyederförmigen Partikeln vor. Bezüglich möglicher nicht-polyederförmiger Ausbildungen der Partikel wird auf die im Zusammenhang der osteokonduktiven Stützkörper beschriebenen Nicht-Polyederformen Bezug genommen.

In einer weiteren Ausführungsform weist der Zusatzstoff oligopodenförmige Partikel auf oder liegt in Form von oligopodenförmigen Partikeln vor. Bezüglich möglicher oligopodenförmiger Ausbildungen der Partikel wird auf die im Zusammenhang der osteokonduktiven Stützkörper beschriebenen Oligopodenformen Bezug genommen.

Der Zusatzstoff ist vorzugsweise ausgewählt aus der Gruppe bestehend aus osteoaktiver Stoff, antimikrobielle Substanz wie Antibiotikum, entzündungshemmendes Arzneimittel, Zytostatikum, Zytokin, knochenmorphogenetisches Protein (Bone Morphogenetic Protein, BMP), Arzneimittel gegen Osteoporose, Hyaluronsäure, Kontrastmittel und Mischungen aus wenigstens zwei der genannten Zusatzstoffe.

Ein osteoaktiver Stoff ist als Zusatzstoff besonders bevorzugt. Durch einen solchen Zusatzstoff kann mit besonderem Vorteil die Neogenese von Knochengewebe stimuliert werden, was den Heilungsprozess eines Knochendefekts zusätzlich unterstützt. Ein osteoaktiver Stoff hat insbesondere den Vorteil, dass Osteoblasten in das Implantat, insbesondere in eine durch die Stützkörper nach ihrer Verdichtung gebildeten Struktur, einwachsen und Osteoiden bilden können, welche anschließend mineralisieren und zu Knochen werden.

Bei dem osteoaktiven Stoff handelt es sich vorzugsweise um einen osteogenen und/oder osteoinduktiven Stoff.

Weiterhin ist es bevorzugt, wenn der osteoaktive Stoff in vivo abbaubar oder in vivo resorbierbar ist.

Der osteoaktive Stoff kann insbesondere eine in vivo Abbauzeit (Degradationszeit) von 1 Tag bis 2 Jahren, insbesondere 3 Tagen bis 10 Monaten, bevorzugt 1 Woche bis 6 Monaten, aufweisen.

Weiterhin kann der osteoaktive Stoff insbesondere eine in vivo Resorptionszeit von 1 Stunde bis 1 Jahr, insbesondere 12 Stunden bis 6 Monaten, bevorzugt 3 Tagen bis 3 Monaten, aufweisen.

Weiterhin kann es sich bei dem osteoaktiven Stoff um einen flexiblen, insbesondere weichen, Stoff handeln.

Bevorzugt ist der osteoaktive Stoff flexibler, insbesondere weicher, als die osteokonduktiven Stützkörper ausgebildet.

Bevorzugt steht der osteoaktive Zusatzstoff nach Implantation in direktem Kontakt zu einem frisch angerauten, blutenden Knochenlager eines zu behandelnden Knochendefekts, bevorzugt eines Azetabulums, und bietet somit gute Voraussetzungen, dass Osteoblasten einwachsen können. Diese können Osteoiden bilden, welche mineralisieren und zu Knochen werden.

In einer weiteren Ausführungsform weist der osteoaktive Stoff einen Anteil von 5 Gew.-% bis 70 Gew.-%, insbesondere 10 Gew.-% bis 50 Gew.-%, bevorzugt 15 Gew.-% bis 25 Gew.-%, auf, bezogen auf das Gesamtgewicht des Implantats.

Der osteaoaktive Zusatzstoff ist vorzugsweise ausgewählt aus der Gruppe bestehend aus Protein wie extrazelluläres Protein, Polysaccharid wie Mucopolysaccharid und/oder Cellulosederivat, biologisches Gewebe, aufbereitetes oder gereinigtes biologisches Gewebe, extrazelluläre Matrix, Polytrimethylencarbonat, Poly-para-dioxanon (Poly-1,4-dioxan-2-on), Polyhydroxyalkanoat, Metall und Mischungen aus wenigstens zwei der genannten Zusatzstoffe.

Das Protein kann insbesondere ausgewählt sein aus der Gruppe bestehend aus Collagen, Gelatine, Elastin, Retikulin, Fibronektin, Fibrin, Laminin, Albumin und Mischungen aus wenigstens zwei der genannten Proteine. Bei dem Collagen handelt es sich vorzugsweise um Collagen Typ I, Collagen Typ III oder um eine Mischung enthaltend oder bestehend aus Collagen Typ I und Collagen Typ III.

Das Polysaccharid kann insbesondere ausgewählt sein aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Carboxyethylcellulose, Chitin, Chitosan, Hyaluronsäure, Dextransulfat, Heparin, Heparansulfat, Chondroitinsulfat wie Chondroitin-4-sulfat und/oder Chondroitin-6-sulfat, Dermatansulfat, Keratansulfat und Mischungen aus wenigstens zwei der genannten Polysaccharide.

Bei dem biologischen Gewebe kann es sich insbesondere um ein tierisches oder xenogenes, bevorzugt bovines, equines oder porcines, Gewebe handeln.

Das Gewebe kann grundsätzlich ausgewählt sein aus der Gruppe bestehend aus Perikard, Peritoneum, Dünndarm-Submukosa, Magen-Submukosa, Harnblasen-Submukosa, Uterus-Submukosa, Serosa und Mischungen aus wenigstens zwei der genannten Gewebe.

Bevorzugt ist das Gewebe ausgewählt aus der Gruppe bestehend aus Perikard (Herzbeutel), Pericardium fibrosum, Pericardium serosum, Epikard, Plattenepithel, Tunica serosa, Muskel wie beispielsweise Myokard und Mischungen aus wenigstens zwei der genannten Gewebe.

Besonders bevorzugt handelt es sich bei dem Gewebe um Perikard, insbesondere um bovines Perikard, d.h. um Rinderperikard.

Bei dem aufbereiteten bzw. gereinigten biologischen Gewebe kann es sich insbesondere um ein von nichtcollagenen Bestandteilen, bevorzugt Fetten und/oder Enzymen und/oder nichtcollagenen Proteinen, befreites biologisches Gewebe handeln. Besonders bevorzugt handelt es sich bei dem aufbereiteten bzw. gereinigten Gewebe um ein aus Rinderperikard hergestelltes und von nichtcollagenen Bestandteilen, insbesondere Fetten, Enzymen und nichtcollagenen Proteinen, gereinigtes und lyophilisiertes Collagenmaterial. Ein derartiges Material wird von der Anmelderin unter der Bezeichnung Lyoplant^{®} bereits kommerziell für den Duraersatz angeboten. Bezüglich weiterer Merkmale des Gewebes wird auf die vorangegangenen Ausführungen Bezug genommen.

Bei der extrazellulären Matrix kann es sich insbesondere um eine extrazelluläre Matrix eines biologischen Gewebes handeln. Bevorzugt handelt es sich bei der extrazellulären Matrix um die extrazelluläre Matrix eines tierischen, insbesondere bovinen, equinen oder procinen, Gewebes. Bezüglich weiterer Merkmale des Gewebes wird ebenfalls auf die vorangegangenen Ausführungen Bezug genommen.

Das Polyhydroxyalkanoat kann insbesondere ausgewählt sein aus der Gruppe bestehend aus Polyglycolid, Polylactid, Polycaprolacton, Poly-3-hydroxybutyrat, Poly-4-hydroxybutyrat, Copolymere aus wenigstens zwei der genannten Polymere und Mischungen (Blends) aus wenigstens zwei der genannten Polymere.

Bei dem Metall kann es sich insbesondere um Titan und/oder Tantal handeln.

In einer weiteren Ausführungsform liegt der osteoaktive Zusatzstoff in unvernetzter Form vor.

In einer weiteren Ausführungsform liegt der osteoaktive Zusatzstoff in vernetzter Form vor. Beispielsweise kann es sich bei dem osteoaktiven Zusatzstoff um ein vernetztes Protein, insbesondere vernetztes Collagen oder vernetzte Gelatine, handeln.

Das im Zusammenhang des Zusatzstoffes genannte Zytokin kann insbesondere ausgewählt sein aus der Gruppe bestehend aus Interferone, Interleukine wie Interleukin-1β und/oder Interleukin-6, koloniestimulierende Faktoren, Chemokine und Mischungen aus wenigstens zwei der genannten Zytokine.

Das im Zusammenhang des Zusatzstoffes genannte knochenmorphogenetische Protein kann insbesondere ausgewählt sein aus der Gruppe bestehend aus BMP 1, BMP 2, BMP 3, BMP 3B, BMP 4, BMP 5, BMP 6, BMP 7, BMP 8A, BMP 8B, BMP 10, BMP 15, und Mischungen aus wenigstens zwei der genannten knochenmorphogenetischen Proteine.

Die im Zusammenhang des Zusatzstoffes genannte antimikrobielle Substanz kann insbesondere ausgewählt sein aus der Gruppe bestehend Polyhexamethylenbiguanid (PHMB), Silber, Silberverbindungen, insbesondere Silbersalze, bevorzugt in Form von Nanopartikeln, und Mischungen aus wenigstens zwei der genannten antimikrobiellen Substanzen.

Bei dem im Zusammenhang des Zusatzstoffes genannten Antibiotikum kann es sich insbesondere um Gentamycin handeln.

Das im Zusammenhang des Zusatzstoffes genannte Kontrastmittel kann insbesondere ausgewählt sein aus der Gruppe bestehend aus Jodverbindungen, Schwermetallsalze wie Bariumsulfat, Zirkoniumoxid und Mischungen aus wenigstens zwei der genannten Kontrastmittel.

In einer weiteren Ausführungsform handelt es sich bei dem Implantat um ein Implantat zur Verwendung bei der Behandlung und/oder biologischen Rekonstruktion, insbesondere Auskleidung und/oder Abdichtung und/oder Unterfütterung und/oder wenigstens teilweisen Auffüllung, eines Knochendefekts.

Wie bereits erwähnt, kann es sich bei dem Knochendefekt insbesondere um einen Gelenksdefekt, bevorzugt Hüftgelenksdefekt, besonders bevorzugt Azetabulumdefekt, handeln.

In einer weiteren Ausführungsform handelt es sich bei der Behandlung um eine Revision.

In einer weiteren Ausführungsform handelt es sich bei dem Implantat um ein Knochenimplantat.

In einer weiteren Ausführungsform handelt es sich bei dem Implantat um ein Knochenersatzmaterial.

Weitere Merkmale und Vorteile der Erfindung ergeben sich aus den Ansprüchen sowie aus der nachfolgenden Beschreibung bevorzugter Ausführungsformen anhand von Figurenbeschreibungen und der dazugehörigen Figuren. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### KURZBESCHREIBUNG DER FIGUREN

- Fig. 1:: eine Draufsicht auf ein menschliches Azetabulum,
- Fig. 2:: eine Ausführungsform eines nicht erfindungsgemäßen Implantats,
- Fig. 3:: eine weitere Ausführungsform eines nicht erfindungsgemäßen Implantats,
- Fig. 4:: eine weitere Ausführungsform eines nicht erfindungsgemäßen Implantats,
- Fig. 5:: eine weitere Ausführungsform eines nicht erfindungsgemäßen Implantats,
- Fig. 6a-d:: verschiedene Ausführungsformen von osteokonduktiven Stützkörpern,
- Fig. 7:: eine Ausführungsform von osteokonduktiven Stützkörpern in Kombination mit einem fadenförmigen Zugelement,
- Fig. 8a-c:: Behandlung eines defekten Azetabulums mittels eines nicht erfindungsgemäßen Implantats,
- Fig. 9:: eine nicht erfindungsgemäße Ausführungsform einer Einbringhilfe,
- Fig. 10:: eine weitere Ausführungsform eines nicht erfindungsgemäßen Implantats,
- Fig. 11:: eine weitere Ausführungsform eines nicht erfindungsgemäßen Implantats,
- Fig. 12:: eine weitere Ausführungsform eines nicht erfindungsgemäßen Implantats und
- Fig. 13:: eine weitere Ausführungsform eines nicht erfindungsgemäßen Implantats.

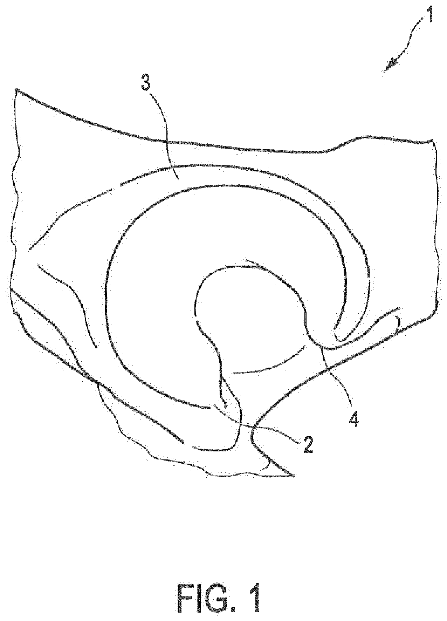

Fig. 1 zeigt schematisch eine Draufsicht auf ein menschliches Azetabulum 1, auch als Hüftgelenk- oder Beckenpfanne bezeichnet. Hierbei handelt es sich um den vom Becken gebildeten knöchernen Anteil des Hüftgelenks. Das Azetabulum entsteht durch Verschmelzung von Teilen des Sitz-, Darm- und Schambeins. Die Verschmelzung ist mit ca. 6 Monaten abgeschlossen.

Bei idealen Voraussetzungen besteht eine Übereinstimmung zwischen Azetabulum und Femurkopf, d.h. der runde Femurkopf passt genau in das Azetabulum, welches ihn weitgreifend einbettet und umschließt. Das Hüftgelenk ist als vielachsiges Kugelgelenk ausgebildet und dadurch in nahezu jede Richtung mehr oder weniger frei beweglich. Dies gewährleistet eine hohe Beweglichkeit und Belastbarkeit.

Die gelenkbildenden Anteile des Hüftgelenks sind von einer bindegewebigen Kapsel umgeben, deren Innenschicht, die Synovia, ständig neue Gelenkflüssigkeit produziert. Eine ringförmige Gelenklippe aus Knorpel bildet den Rand der knöchernen Pfanne.

Das Azetabulum 1 besitzt einen vorderen Pfannenrand 2, auch als sogenanntes Vorderhorn bezeichnet, sowie einen hinteren Pfannenrand 4, auch als sogenanntes Hinterhorn bezeichnet. Das dazwischenliegende Pfannendach 3 ist rund oder im Wesentlichen rund auslaufend ausgebildet.

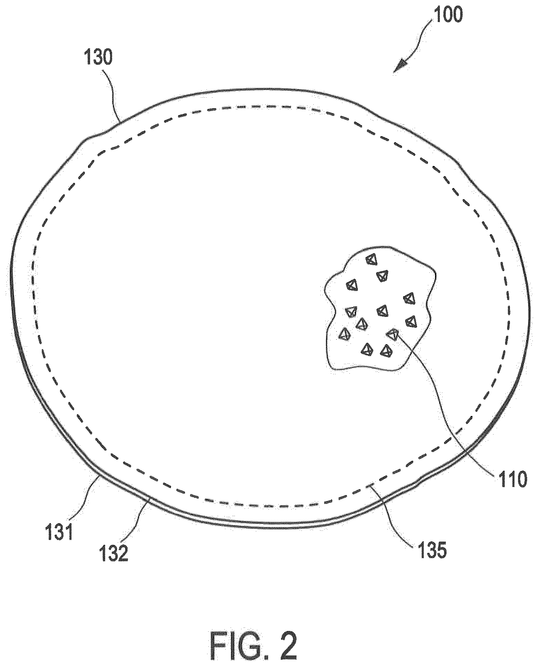

Fig. 2 zeigt schematisch eine Ausführungsform eines nicht erfindungsgemäßen Implantats 100.

Das Implantat 100 weist osteokonduktive Stützkörper 110 sowie eine Einbringhilfe 130 auf. Die Einbringhilfe 130 ist als eine die osteokonduktiven Stützkörper 110 umschließende Umhüllung ausgebildet.

Die osteokonduktiven Stützkörper 110 können beispielsweise - wie dargestellt - in Form von Tetraedern vorliegen. Die tetraederförmige Ausgestaltung der Stützköper 110 begünstigt ein Verdichten, insbesondere Impaktieren, der Stützkörper, wodurch eine dreidimensionale Struktur geschaffen werden kann, welche in besonderer Weise die Spongiosa von menschlichem oder tierischem Knochen wiederspiegeln kann.

Die Umhüllung 130 weist zwei übereinander angeordnete Lagen 131; 132 auf, welche randseitig mittels einer Naht 135 miteinander verbunden sind. Dabei können die Lagen 131; 132 - wie dargestellt - beispielsweise in etwa scheibenförmig gestaltet sein. Die Naht 135 kann beispielsweise aus einem in vivo nicht abbaubaren/in vivo nicht resorbierbaren Nahtmaterial, wie beispielsweise einem Polypropylenfaden, oder einem in vivo abbaubaren/in vivo resorbierbaren Nahtmaterial, wie beispielsweise einem Polyglycolidfaden, gebildet sein.

Die beiden miteinander vernähten Lagen 131; 132 schließen einen Hohlraum ein, welcher mit den osteokonduktiven Stützkörpern 110 wenigstens teilweise, insbesondere nur teilweise, befüllt ist.

Die beiden Lagen 131; 132 bestehen bevorzugt aus einem in vivo abbaubaren/in vivo resorbierbaren Material. Bei dem Material kann es sich insbesondere um Collagen, vorzugsweise um ein solches handeln, welche aus Rinderperikard hergestellt ist. Dies hat den Vorteil, dass neues Knochengewebe in das Implantat und mithin den Knochendefekt einwachsen kann.

Die osteokonduktiven Stützkörper 110 wirken mit besonderem Vorteil als Leitstruktur für einwachsendes Knochengewebe.

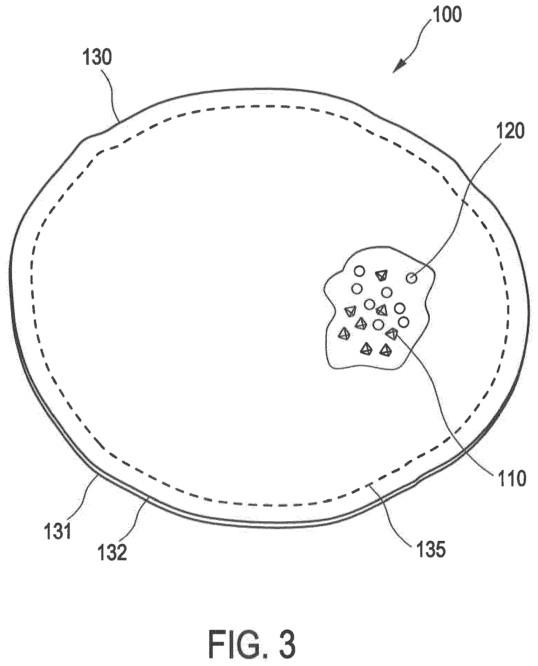

Fig. 3 zeigt schematisch eine weitere Ausführungsform eines nicht erfindungsgemäßen Implantats 100.

Das Implantat 100 weist osteokonduktive Stützkörper 110, einen osteoaktiven Stoff 120, wie beispielsweise Collagen und/oder Fibrin, sowie eine Einbringhilfe 130 auf. Die Einbringhilfe 130 ist als eine die osteokonduktiven Stützkörper 110 sowie den osteoaktiven Stoff 120 umschließende Umhüllung ausgebildet.

Bezüglich weiterer Merkmale und Vorteile des Implantats 100, insbesondere der Einbringhilfe bzw. Umhüllung 130, wird vollständig auf die zur Figur 2 gemachten Ausführungen Bezug genommen.

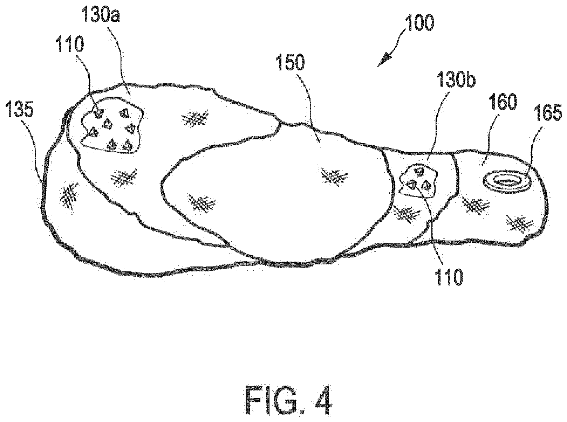

Fig. 4 zeigt schematisch eine weitere Ausführungsform eines nicht erfindungsgemäßen Implantats 100.

Das Implantat 100 weist osteokonduktive Stützkörper 110 sowie zwei Einbringhilfen 130a und 130b auf, welche jeweils als Umhüllungen ausgebildet sind. In beiden Umhüllungen 130a; 130b sind jeweils osteokonduktive Stützkörper 110 enthalten.

Das Implantat weist weiterhin einen Zwischenbereich 150 sowie einen Lateralbereich 160 auf.

Der Lateralbereich 160 kann eine Befestigungseinrichtung 165, beispielsweise in Form einer Hülse, aufweisen. Die Befestigungseinrichtung 165 ist vorzugsweise dazu ausgebildet, eine Befestigung des Implantats 100 an Knochengewebe zu bewirken, welches vorzugsweise an einen Knochendefekt angrenzt.

Das Implantat 100 kann ferner zur Ausbildung der Umhüllungen 130a; 130b und/oder zur Verstärkung eine Naht 135 aufweisen. Die Naht 135 ist vorzugsweise durchgehend entlang von Randzonen des Implantats 100 und/oder der beiden Umhüllungen 130a; 130b ausgebildet. Die Naht 135 kann von einem schrumpffähigen Faden, wie beispielsweise einem Faden aus Poly-4-hydroxybutyrat, gebildet sein. Dadurch kann beispielsweise durch eine strahleninduzierte Schrumpfung der Naht 135 mit besonderem Vorteil eine Formanpassung des Implantats 100 an eine patientenspezifische Form eines Knochendefekts erzielt werden.

Sowohl die beiden Umhüllungen 130a; 130b als auch die Abschnitte 150 und 160 können jeweils eine Netzstruktur, insbesondere eine gewirkte Netzstruktur, beispielsweise mit monofilen Polypropylenfäden, besitzen.

Bezüglich weiterer Merkmale und Vorteile des dargestellten Implantats 100 wird auf die im Zusammenhang der Figur 2 gemachten Ausführungen Bezug genommen.

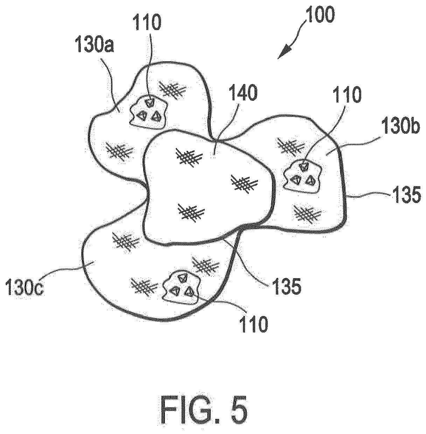

Fig. 5 zeigt schematisch eine weitere Ausführungsform eines nicht erfindungsgemäßen Implantats 100.

Das Implantat 100 weist einen Zentralbereich 140 sowie drei als Umhüllungen ausgebildete Einbringhilfen 130a; 130b; 130c auf.

Die Umhüllungen 130a; 130b; 130c sind radial um den Zentralbereich 140 angeordnet.

Die Umhüllungen 130a; 130b; 130c sind jeweils wenigstens teilweise, insbesondere nur teilweise, mit osteokonduktiven Stützkörpern 110 befüllt.

Der Zentralbereich 140 ist vorzugsweise zur Anlage an einen Bodenbereich eines Knochendefekts ausgebildet, während die Umhüllungen 130a; 130b; 130c vorzugsweise zur Anlage an Seitenwände, insbesondere an einen knöchernen Defektboden radial umlaufende Knochenwände, ausgebildet sind.

Sowohl die Umhüllungen 130a; 130b; 130c als auch der Zentralbereich 140 weisen jeweils eine randseitig verlaufende Naht 135 auf. Auch in diesem Fall kann die Naht 135 beispielsweise von einem Schrumpffaden, wie beispielsweise einem Faden aus Poly-4-hydroxybutyrat, gebildet sind. Durch eine gezielte Schrumpfung der Naht 135, beispielsweise mittels Bestrahlen, kann eine Anlage der Umhüllungen 130a; 130b; 130c an einen Knochendefektboden radial umlaufende Knochenwände erleichtert werden.

Die Umhüllungen 130a; 130b; 130c sowie der Zentralbereich 140 können jeweils zwei übereinander angeordnete Netzlagen, insbesondere gewirkte Netzlagen, aufweisen, welche über die Naht 135 randseitig miteinander verbunden sind.

Dadurch definieren die Umhüllungen 130a; 130b; 130c sowie der Zentralbereich 140 jeweils Hohlräume. Wenigstens die Hohlräume der Umhüllungen 130a; 130b; 130c können dabei jeweils wenigstens teilweise, insbesondere nur teilweise, mit den osteokonduktiven Stützkörpern 110 befüllt sein.

Das Implantat 100 ist insgesamt vorzugsweise nach Art eines dreiflügeligen Propellers ausgestaltet, wobei der Zentralbereich 140 die "Welle" und die Umhüllungen 130a; 130b; 130c die "Flügel" des Propellers bilden.

Bezüglich weiterer Merkmale und Vorteile des in Figur 5 dargestellten Implantats 100 wird auf die im Zusammenhang der Figur 2 gemachten Ausführungen Bezug genommen.

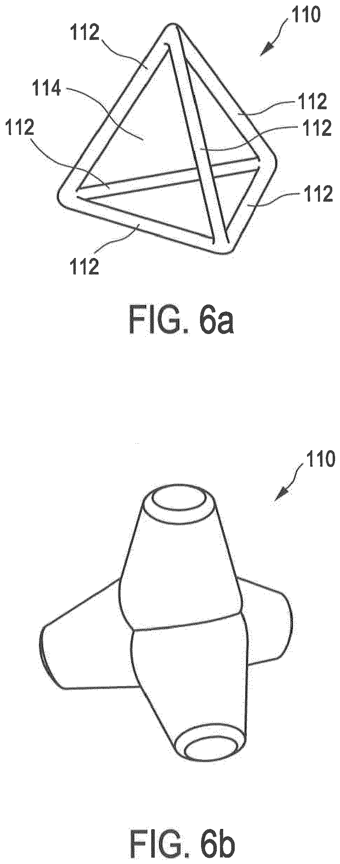

Fig. 6a-d zeigen jeweils eine Ausführungsform für die osteokonduktiven Stützkörper 110, welche jeweils ein Verdichten, insbesondere Impaktieren, beispielsweise mittels eines Nachschlägers, begünstigen.

Der in Figur 6a dargestellte Stützkörper 110 weist längliche und geradlinig verlaufende Strukturelemente 112 auf, welche zu einer tetraederförmigen Gesamtstruktur zusammengesetzt sind. Das durch die gegenseitige Anordnung der Strukturelemente 112 erzeugte Hohlraumvolumen 114 trägt in besonders vorteilhafter Weise zu einer Erhöhung des absoluten Hohlraumraumvolumens einer dreidimensionalen und osteokonduktiven Struktur, welche durch Verdichten, insbesondere Impaktieren, der osteokonduktiven Stützkörper erhältlich ist, bei. Dadurch ist es beispielsweise möglich, die Spongiosa von menschlichem oder tierischem Knochen, insbesondere hinsichtlich der Porosität, nachzuahmen.

Obendrein begünstigt die in Figur 6a dargestellte Ausführungsform ein Ineinandergreifen, insbesondere ein gegenseitiges Einklemmen, der osteokonduktiven Stützkörper bei Kraftbeaufschlagung und mithin die Erzeugung einer durch die Stützkörper gebildeten Leitstruktur.

Der in Figur 6b dargestellte Stützkörper 110 liegt in Form eines Tetrapoden vor. Eine tetrapodenförmige Ausgestaltung der Stützkörper begünstigt ebenfalls ein Ineinandergreifen, insbesondere ein gegenseitiges Einklemmen, der osteokonduktiven Stützkörper bei Kraftbeaufschlagung und mithin die Erzeugung einer durch die Stützkörper gebildeten Leitstruktur.

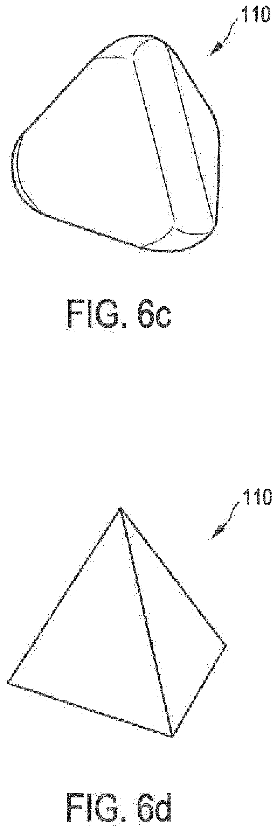

Der in Figur 6c dargestellte Stützkörper 110 liegt in Form eines Tetraeders vor. Eine tetraederförmige Ausgestaltung der Stützkörper begünstigt ebenfalls ein Ineinandergreifen, insbesondere ein gegenseitiges Verkeilen, der osteokonduktiven Stützkörper bei Kraftbeaufschlagung und mithin die Erzeugung einer durch die Stützkörper gebildeten Leitstruktur.

Der in Figur 6d dargestellte Stützkörper 110 liegt in Form einer Pyramide vor. Eine pyramidenförmige Ausgestaltung der Stützkörper begünstigt ebenfalls ein Ineinandergreifen, insbesondere ein gegenseitiges Verkeilen, der osteokonduktiven Stützkörper bei Kraftbeaufschlagung und mithin die Erzeugung einer durch die Stützkörper gebildeten Leitstruktur.

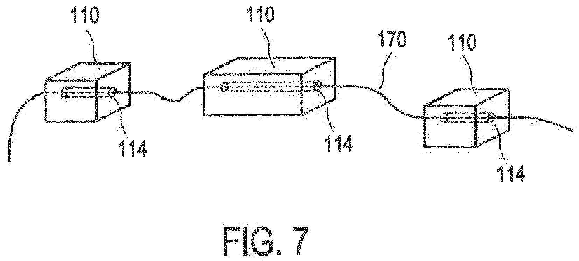

Fig. 7 zeigt schematisch osteokonduktive Stützkörper 110 sowie ein längliches Zugelement 170, wie sie im Rahmen der vorliegenden Erfindung zum Einsatz kommen können.

Die Stützkörper 110 besitzen jeweils durchgehende Öffnungen 114 und können - wie dargestellt - beispielsweise quaderförmig ausgebildet sein. Durch die Öffnungen 114 kann das längliche Zugelement 170, wie dargestellt, hindurchgeführt werden. Auf diese Weise ist ein Kompaktieren, insbesondere Verzurren, der Stützkörper 110 unter Ausbildung osteokonduktiven Leitstruktur erzielbar. Bei dem Zugelement 170 handelt es sich vorzugsweise um einen Faden, beispielsweise aus Polypropylen oder einem Polyhydroxyalkanoat, wie beispielsweise Polylactid oder Polyglykolid.

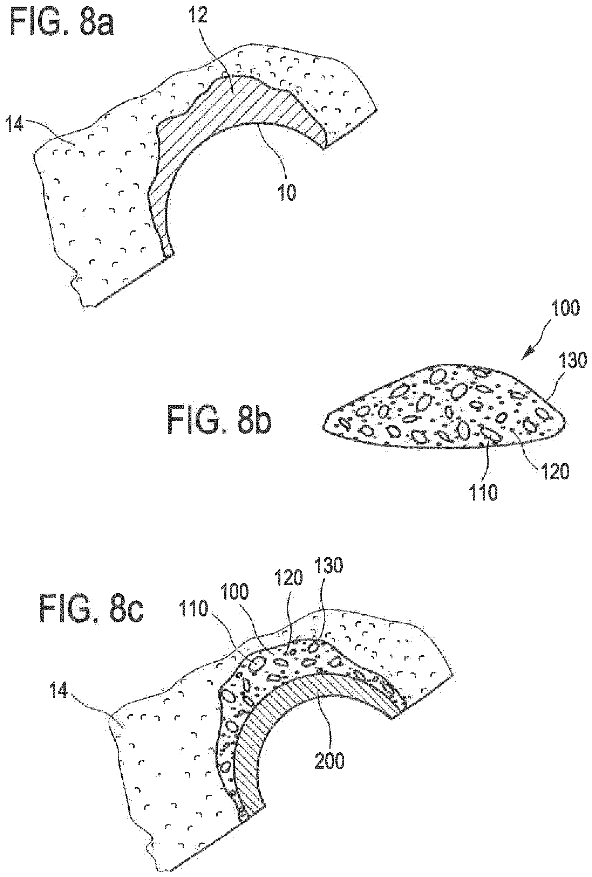

In den Fig. 8a-c ist schematisch die Behandlung eines defekten Azetabulums mittels eines nicht erfindungsgemäßen Implantats dargestellt.

Fig. 8a zeigt ein Azetabulum 10 mit einem Defekt 12 sowie umliegendes Knochengewebe 14.

Das Implantat 100 weist osteokonduktive Stützkörper 110, einen osteoaktiven Stoff 120 sowie eine Einbringhilfe 130 auf. Die Einbringhilfe 130 ist als eine die osteokonduktiven Stützkörper 110 und den osteoaktiven Stoff 120 umgebende Umhüllung ausgebildet (siehe Fig. 8b).

Zunächst wird das Implantat 100 in das defekte Azetabulum 10 platziert.

Nach Platzieren des Implantats 100 werden die osteokonduktiven Stützkörper 110 vorzugsweise in einen verdichteten, insbesondere impaktierten, Zustand überführt. Dies kann beispielsweise mittels eines sogenannten Nachschlägers erfolgen.

Anschließend wird eine künstliche Gelenkpfanne 200 auf das Implantat 100 oder auf einen gegebenenfalls zuvor auf das Implantat 100 zusätzlich aufgebrachten Knochenzement implantiert (siehe Fig. 8c)

Ist die Umhüllung 130 in vivo abbaubar oder in vivo resorbierbar und/oder offenporig ausgebildet, kommt es vorteilhafterweise bereits innerhalb der ersten vier Wochen nach dem operativen Eingriff zu einem Einwachsen von Knochengewebe, insbesondere Knochenneugewebe, in das Implantat 100 und mithin in das defekte Azetabulum 10.

Dabei wirken die vorzugsweise verdichteten, insbesondere impaktierten, Stützkörper 110 des Implantats 100 als osteokonduktive Leitstruktur für das einwachsende Knochengewebe, während der osteoaktive Stoff 120 mit besonderem Vorteil das Einwachsen von Knochengewebe verstärkt und/oder stimuliert. Auf diese Weise kann das Implantat 100 eine effektive Erhöhung der Sekundärstabilität der implantierten Gelenkpfanne 200 bewirken.

Bezüglich weiterer Merkmale und Vorteile des Implantats 100 wird auf die im Zusammenhang der Figur 2 gemachten Ausführungen Bezug genommen.

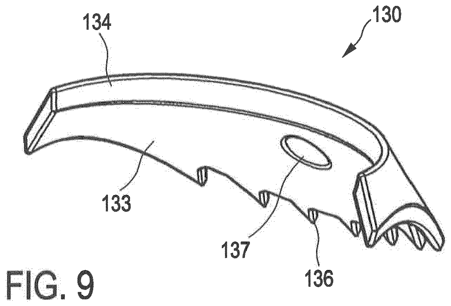

Fig. 9 zeigt schematisch eine als plattenförmige Abdeckung ausgebildete Einbringhilfe 130.

Die Abdeckung 130 weist eine konkave Fläche 133 auf, an welcher osteokonduktive Stützkörper fixiert bzw. befestigt werden können.

Weiterhin weist die Abdeckung 130 einen konkaven Verbindungsbereich 134 auf. Der Verbindungsbereich 134 ist dazu ausgebildet, eine Verbindung mit einer künstlichen Gelenkpfanne, insbesondere mit einer konvexen Oberfläche einer künstlichen Gelenkpfanne, zu bewirken.

Des Weiteren weist die Abdeckung 130 Verankerungsstifte 136 auf. Die Verankerungsstifte 136 können in einen Knochen eingeschlagen werden, wodurch die Abdeckung 130 im Bereich eines zu behandelnden Knochendefekts verankert werden kann.

Ferner weist die Abdeckung 130 eine Befestigungsmittel-Öffnung 137 auf. Die Öffnung 137 ist zur Aufnahme eines Befestigungsmittels, wie beispielsweise einer Verschlussschraube, ausgebildet. Dadurch wird eine zusätzliche Verankerungsmöglichkeit der Abdeckung 130 geschaffen.

Sowohl die Verankerungsstifte 136 als auch die Befestigungsmittel-Öffnung 137 dienen der Erzielung einer Primärstabilität.

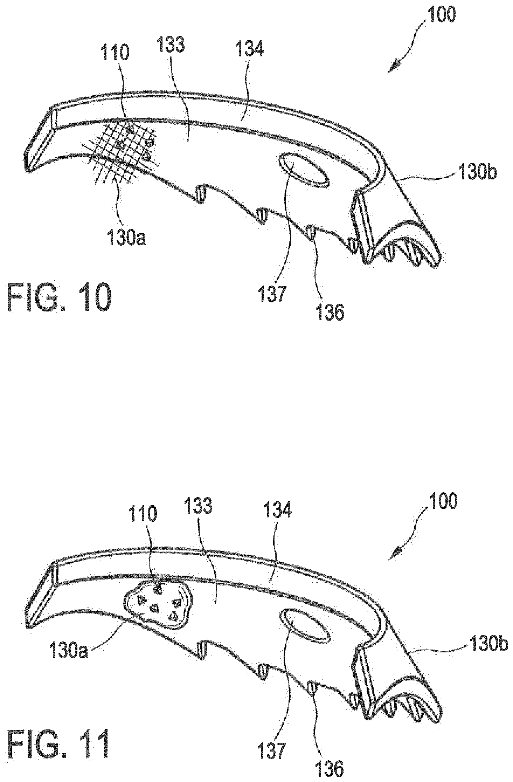

Fig. 10 stellt schematisch eine weitere Ausführungsform eines nicht erfindungsgemäßen Implantats 100 dar.

Das Implantat 100 weist osteokonduktive Stützkörper 110 sowie eine als Kombination aus einem Netz 130a und einer plattenförmigen Abdeckung 130b ausgebildete Einbringhilfe auf.

Das Netz 130a fixiert die osteokonduktiven Stützkörper 110 vorzugsweise an einer konkaven Fläche 133 der Abdeckung 130b. Hierzu ist das Netz 130a vorzugsweise stoffschlüssig mit der Fläche 133 verbunden. Beispielsweise kann das Netz 130a mit der Fläche 133 verklebt sein.

Bei dem Netz 130a kann es sich weiterhin um ein gewirktes Netz, insbesondere um ein gewirktes Polypropylennetz, handeln.

Bezüglich weiterer Merkmale und Vorteile der Abdeckung 130b wird vollständig auf die im Zusammenhang der Figur 9 gemachten Ausführungen Bezug genommen. Die dort in Bezug auf die Abdeckung 130 gemachten Ausführungen gelten sinngemäß auch für die in Figur 10 dargestellte Abdeckung 130b.

Fig. 11 zeigt eine weitere Ausführungsform eines nicht erfindungsgemäßen Implantats 100.

Das Implantat 100 weist osteokonduktive Stützkörper 110 sowie eine als Kombination aus einem Bindemittel 130a und einer plattenförmigen Abdeckung 130b ausgebildete Einbringhilfe auf.

Das Bindemittel 130a verbindet, vorzugsweise verklebt, die osteokonduktiven Stützkörper 110 miteinander. Gleichzeitig fixiert das Bindemittel 130a die osteokonduktiven Stützkörper 110 vorzugsweise an einer konkaven Fläche 133 der Abdeckung 130b.

Das Bindemittel 130a weist vorzugsweise ein Protein, insbesondere Collagen und/oder Gelatine, und/oder ein Polysaccharid, insbesondere ein Cellulosederivat und/oder Hyaluronsäure, auf.

Bezüglich weiterer Merkmale und Vorteile der Abdeckung 130b wird vollständig auf die im Zusammenhang der Fig. 9 gemachten Ausführungen Bezug genommen. Die dort in Bezug auf die Abdeckung 130 gemachten Ausführungen gelten sinngemäß auch für die in Fig. 11 dargestellte Abdeckung 130b.

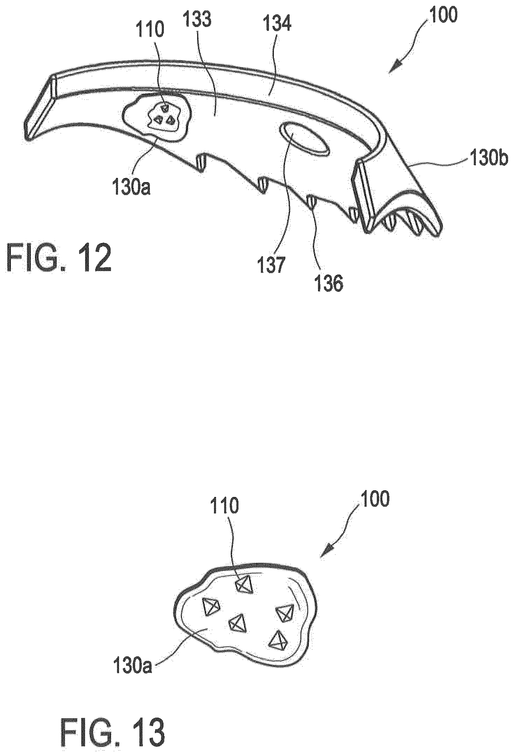

Fig. 12 zeigt eine weitere Ausführungsform eines nicht erfindungsgemäßen Implantats 100.

Das Implantat 100 weist osteokonduktive Stützkörper 110 sowie eine als Kombination aus einer Umhüllung 130a und einer plattenförmigen Abdeckung 130b ausgebildete Einbringhilfe auf.

Die Umhüllung 130a fixiert die osteokonduktiven Stützkörper 110 vorzugsweise an einer konkaven Fläche 133 der Abdeckung 130b. Hierzu ist die Umhüllung 130a vorzugsweise stoffschlüssig mit der Fläche 133 verbunden. Beispielsweise kann die Umhüllung 130a mit der Fläche 133 verklebt sein. Beispielsweise kann hierzu das in Fig. 11 beschriebene Bindemittel verwendet werden.

Die Umhüllung 130a kann netzförmig ausgestaltet oder aus einer tierischen Membran, vorzugsweise aus Perikard, hergestellt sein.

Bezüglich weiterer Merkmale und Vorteile der Abdeckung 130b wird vollständig auf die im Zusammenhang der Fig. 9 gemachten Ausführungen Bezug genommen. Die dort in Bezug auf die Abdeckung 130 gemachten Ausführungen gelten sinngemäß auch für die in Fig. 12 dargestellte Abdeckung 130b.

Fig. 13 zeigt schematisch eine weitere Ausführungsform eines nicht erfindungsgemäßen Implantats 100.

Das Implantat 100 weist osteokonduktive Stützkörper 110 sowie eine als Bindemittel ausgebildete Einbringhilfe 130 auf.

Das Bindemittel 130 verbindet, vorzugsweise verklebt, die osteokonduktiven Stützkörper 110 miteinander.

Das dargestellte Implantat 100 kann mit besonderem Vorteil während eines operativen Eingriffs leicht in Bezug auf Form und Menge an einen zu behandelnden Knochendefekt angepasst werden.

Bezüglich weiterer Merkmale und Vorteile der in den Figuren dargestellten Implantate wird vollständig auf die allgemeine Beschreibung Bezug genommen.

## Patentansprüche

1. Implantat, insbesondere zum Behandeln eines Knochendefekts, aufweisend:
- osteokonduktive Stützkörper und
- eine Einbringhilfe,
wobei die Einbringhilfe zum Einbringen der osteokonduktiven Stützkörper in einen Knochendefekt und zum Zusammenhalten der osteokonduktiven Stützkörper ausgebildet ist, wobei die Einbringhilfe als Kombination aus einem textilen Flächengebilde und einem Bindemittel ausgebildet ist, **dadurch gekennzeichnet, dass** das Bindemittel ein Verdünnungsmittel aufweist und die osteokonduktiven Stützkörper mittels des Bindemittels miteinander unter Ausbildung einer pastösen oder knetbaren Zubereitung verklebt sind.

2. Implantat nach Anspruch 1, **dadurch gekennzeichnet, dass** die osteokonduktiven Stützkörper Apatit und/oder Tricalciumphosphat aufweisen oder aus Apatit und/oder Tricalciumphosphat bestehen.

3. Implantat nach Anspruch 2, **dadurch gekennzeichnet, dass** der Apatit und/oder das Tricalciumphosphat eine Porosität von 1 % bis 50 %, insbesondere 5 % bis 20 %, bevorzugt 10 % bis 15 %, aufweisen.

4. Implantat nach Anspruch 2, **dadurch gekennzeichnet, dass** der Apatit und/oder das Tricalciumphosphat nicht porös ausgebildet sind.

5. Implantat nach einem der Ansprüche 2 bis 4, **dadurch gekennzeichnet, dass** der Apatit ausgewählt ist aus der Gruppe bestehend aus Hydroxylapatit, Fluorapatit, Chlorapatit, Carbonat-Fluor-Apatit und Mischungen aus wenigstens zwei der genannten Apatite.

6. Implantat nach einem der Ansprüche 2 bis 5, **dadurch gekennzeichnet, dass** das Tricalciumphosphat ausgewählt ist aus der Gruppe bestehend aus alpha-Tricalciumphosphat, beta-Tricalciumphosphat und eine Mischung aus alpha-Tricalciumphosphat und beta-Tricalciumphosphat.

7. Implantat nach einem der vorhergehenden Ansprüche, **dadurch gekennzeichnet, dass** es sich bei dem textilen Flächengebilde um ein Netz, insbesondere gewirktes Netz, handelt.

8. Implantat nach einem der vorhergehenden Ansprüche, **dadurch gekennzeichnet, dass** das Bindemittel ein Protein, insbesondere Kollagen und/oder Gelatine, und/oder ein Polysaccharid, insbesondere Cellulosederivat wie Carboxymethycellulose und/oder Hyaluronsäure, aufweist.

9. Implantat nach einem der vorhergehenden Ansprüche, **dadurch gekennzeichnet, dass** es sich bei dem Verdünnungsmittel um Glycerin handelt.

10. Implantat nach einem der vorhergehenden Ansprüche, **dadurch gekennzeichnet, dass** das Bindemittel einen Anteil an flüssigem Verdünnungsmittel, vorzugsweise Glycerin und/oder Wasser, von 50 Gew.-% bis 95 Gew.-%, insbesondere 60 Gew.-% bis 90 Gew.-%, bevorzugt 70 Gew.-% bis 80 Gew.-%, aufweist, bezogen auf das Gesamtgewicht des Bindemittels.

11. Implantat nach einem der vorhergehenden Ansprüche, **dadurch gekennzeichnet, dass** es sich bei dem Bindemittel um ein wasserfreies Bindemittel handelt.

12. Implantat nach einem der Ansprüche 1 bis 10, **dadurch gekennzeichnet, dass** das Bindemittel einen Wassergehalt kleiner 5 Gew.-%, insbesondere kleiner 3 Gew.-%, bevorzugt kleiner 1 Gew.-%, aufweist, bezogen auf das Gesamtgewicht des Bindemittels.

13. Implantat nach Anspruch 11 oder 12, **dadurch gekennzeichnet, dass** das Bindemittel Carboxymethylcellulose und Glycerin aufweist.

## Claims

1. Implant, especially for the treatment of a bone defect, comprising:
- osteoconductive supporting bodies and
- an insertion aid,
the insertion aid being designed for insertion of the osteoconductive supporting bodies into a bone defect and for holding together the osteoconductive supporting bodies, the insertion aid being designed as a combination of a textile fabric and a binder, **characterized in that** the binder comprises a diluent and the osteoconductive supporting bodies are bonded together by means of the binder to form a paste-like or kneadable preparation.

2. Implant according to Claim 1, **characterized in that** the osteoconductive supporting bodies comprise apatite and/or tricalcium phosphate or consist of apatite and/or tricalcium phosphate.

3. Implant according to Claim 2, **characterized in that** the apatite and/or the tricalcium phosphate have a porosity of from 1% to 50%, especially 5% to 20%, preferably 10% to 15%.

4. Implant according to Claim 2, **characterized in that** the apatite and/or the tricalcium phosphate are nonporous in form.

5. Implant according to any of Claims 2 to 4, **characterized in that** the apatite is selected from the group consisting of hydroxyapatite, fluorapatite, chlorapatite, carbonate-fluorapatite and mixtures of at least two of said apatites.

6. Implant according to any of Claims 2 to 5, **characterized in that** the tricalcium phosphate is selected from the group consisting of alpha-tricalcium phosphate, beta-tricalcium phosphate and a mixture of alpha-tricalcium phosphate and beta-tricalcium phosphate.

7. Implant according to any of the preceding claims, **characterized in that** the textile fabric is a mesh, especially knitted mesh.

8. Implant according to any of the preceding claims, **characterized in that** the binder comprises a protein, especially collagen and/or gelatin, and/or a polysaccharide, especially a cellulose derivative such as carboxymethyl cellulose and/or hyaluronic acid.

9. Implant according to any of the preceding claims, **characterized in that** the diluent is glycerol.

10. Implant according to any of the preceding claims, **characterized in that** the binder has a content of liquid diluent, preferably glycerol and/or water, of 50% by weight to 95% by weight, especially 60% by weight to 90% by weight, preferably 70% by weight to 80% by weight, based on the total weight of the binder.

11. Implant according to any of the preceding claims, **characterized in that** the binder is an anhydrous binder.

12. Implant according to any of Claims 1 to 10, **characterized in that** the binder has a water content of less than 5% by weight, especially less than 3% by weight, preferably less than 1% by weight, based on the total weight of the binder.

13. Implant according to Claim 11 or 12, **characterized in that** the binder comprises carboxymethyl cellulose and glycerol.

## Revendications

1. Implant, destiné notamment au traitement d'un défaut osseux, ledit implant comportant :
- des corps de support ostéoconducteurs et
- un auxiliaire d'insertion,
l'auxiliaire d'insertion étant conçu pour insérer le corps de support ostéoconducteur dans un défaut osseux et pour maintenir ensemble les corps de support ostéoconducteurs, l'auxiliaire d'insertion étant conçu comme une combinaison d'un tissu textile et d'un liant, **caractérisé en ce que** le liant contient un diluant et les corps de support ostéoconducteurs sont liés les uns aux autres au moyen du liant en formant une préparation pâteuse ou pétrissable.

2. Implant selon la revendication 1, **caractérisé en ce que** les corps de support ostéoconducteurs comprennent de l'apatite et/ou du phosphate tricalcique ou sont en apatite et/ou en phosphate tricalcique.

3. Implant selon la revendication 2, **caractérisé en ce que** l'apatite et/ou le phosphate tricalcique présentent une porosité de 1 % à 50 %, notamment de 5 % à 20 %, de préférence de 10 % à 15 %.

4. Implant selon la revendication 2, **caractérisé en ce que** l'apatite et/ou le phosphate tricalcique sont non poreux.

5. Implant selon l'une des revendications 2 à 4, **caractérisé en ce que** l'apatite est choisie dans le groupe comprenant l'hydroxyapatite, la fluorapatite, la chlorapatite, la carbonate-fluorapatite et des mélanges d'au moins deux des apatites mentionnées.

6. Implant selon l'une des revendications 2 à 5, **caractérisé en ce que** le phosphate tricalcique est choisi dans le groupe comprenant le phosphate tricalcique alpha, le phosphate tricalcique bêta et un mélange de phosphate tricalcique alpha et de phosphate tricalcique bêta.

7. Implant selon l'une des revendications précédentes, **caractérisé en ce que** le tissu textile est un filet, notamment un filet tricoté.

8. Implant selon l'une des revendications précédentes, **caractérisé en ce que** le liant comporte une protéine, notamment du collagène et/ou de la gélatine, et/ou un polysaccharide, notamment un dérivé cellulosique tel que la carboxyméthylcellulose et/ou l'acide hyaluronique.

9. Implant selon l'une des revendications précédentes, **caractérisé en ce que** le diluant est le glycérol.

10. Implant selon l'une des revendications précédentes, **caractérisé en ce que** le liant comporte une proportion de diluant liquide, de préférence de glycérol et/ou d'eau, de 50 % en poids à 95 % en poids, notamment de 60 % en poids à 90 % en poids, de préférence de 70 % en poids à 80 % en poids, par rapport au poids total du liant.

11. Implant selon l'une des revendications précédentes, **caractérisé en ce que** le liant est un liant anhydre.

12. Implant selon l'une des revendications 1 à 10, **caractérisé en ce que** le liant a une teneur en eau inférieure à 5 % en poids, notamment inférieure à 3 % en poids, de préférence inférieure à 1 % en poids, par rapport au total poids du liant.

13. Implant selon la revendication 11 ou 12, **caractérisé en ce que** le liant comporte de la carboxyméthylcellulose et du glycérol.
